# EUROPEAN PATENT SPECIFICATION

(11) **EP 2 041 272 B1**
(45) Date of publication and mention of the grant of the patent: **17.04.2013**
(21) Application number: 07730081.2
(22) Date of filing: 12.06.2007
(51) Int. Cl.: C12N 5/071

(54) **DIFFERENTIATION OF RAT LIVER EPITHELIAL CELLS INTO HEPATOCYTE-LIKE CELLS**
DIFFERENZIERUNG VON RATTENLEBEREPITHELZELLEN ZU HEPATOZYTEN-ÄHNLICHEN ZELLEN
DIFFÉRENTIATION DE CELLULES EPITHELIALES DU FOIE DE RAT EN CELLULES HEPATOCYTAIRES

(30) Priority: 12.06.2006 WO PCT/EP2006/005622
(43) Date of publication of application: 01.04.2009
(73) Proprietor: VRIJE UNIVERSITEIT BRUSSEL, 1050 Brussel (BE)
(72) Inventor: ROGIERS, Vera, 2820 Bonheiden (BE); VANHAECKE, Tamara, 1502 Lembeek (BE); SNYKERS, Sarah, 9340 Lede (BE); PAPELEU, Peggy, 1541 St-pieters-kapelle (BE); VINKEN, Mathieu, 8800 Roeselare (BE); HENKENS, Tom, 9052 Zwijnaarde (BE); DE ROP, Evelien, 2547 Lint (BE); FRACZEK, Joanna, 9000 Gent (BE); DE KOCK, Joery, 2830 Willebroek (BE); DOKTOROVA, Tatyana, 1000 Brussel (BE)
(74) Representative: Michalík, Andrej
(86) International application number: PCT/EP2007/055754
(87) International publication number: WO 2007/144341

(56) References cited:
- WO-A-2006/045331
- GEIMER P ET AL: "THE EPIDERMAL GROWTH FACTOR-INDUCED MIGRATION OF RAT LIVER EPITHELIAL CELLS IS ASSOCIATED WITH A TRANSIENT INHIBITION OF DNA SYNTHESIS" JOURNAL OF CELL SCIENCE, vol. 100, no. 2, 1991, pages 349-356, XP002453245 ISSN: 0021-9533
- SHI XIAO-LEI ET AL: "Hepatocyte-like cells from directed differentiation of mouse bone marrow cells in vitro" ACTA PHARMACOLOGICA SINICA, vol. 26, no. 4, April 2005 (2005-04), pages 469-476, XP002453246 ISSN: 1671-4083
- LESCOAT G ET AL: "MODULATION OF ALPHA FETOPROTEIN ALBUMIN AND TRANSFERRIN GENE EXPRESSION BY CELLULAR INTERACTIONS AND DEXAMETHASONE IN COCULTURES OF FETAL RAT HEPATOCYTES" EUROPEAN JOURNAL OF CELL BIOLOGY, vol. 44, no. 1, 1987, pages 128-134, XP009089869 ISSN: 0171-9335
- SNYKERS S, DE KOCK J, VANHAECKE T, ROGIERS V: "Differentiation of neonatal rat epithelial cells from biliary origin into immature hepatic cells by sequential exposure to hepatogenic cytokines and growth factors reflecting liver development" TOXICOLOGY IN VITRO, 4 April 2007 (2007-04-04), XP002453247

## Description

### FIELD OF THE INVENTION

The present invention relates to methods for cell differentiation, in particular for differentiation of cells to hepatocyte-like cells and in particular departing from cells of a rat liver epithelial cell line. The invention further relates to the so obtained cells and their uses in various pharmacological, toxicological, therapeutic, diagnostic and research applications.

### BACKGROUND OF THE INVENTION

Isolated cells having at least some phenotypic properties of parenchymal hepatocytes can be used in various applications, including but not limited to, cell transplantation, tissue engineering, construction of artificial liver assist devices, testing liver biotransformation of chemical or biological substances, such as drug candidates, testing hepatotoxicity, genotoxic or non-genotoxic carcinogenicity of substances, study the pathogenesis of liver disorders, such as genetic, acquired or infections diseases, among others.

Some of the above or further applications are often performed using cultured primary liver hepatocytes, which may typically be obtained from liver tissue dissected from experimental animals, such as rats, but can also be derived from human donor liver or surgical resection. However, the survival and functionality of primary hepatocytes cultured *in vitro* is often limited, e.g., being less than 1-2 weeks, during which the cells gradually loose their differentiated properties and/or undergo cell death. This may necessitate frequent isolation of primary hepatocytes to ensure sufficient numbers for all applications, with the obvious disposal of large numbers of laboratory animals, because of limited availability of human tissue for experimental purposes. Also a great deal of time and labour are involved.

An object of the present invention is therefore to provide an improved source of cells having useful phenotypic properties of parenchymal hepatocytes, i.e., of hepatocyte-like cells, which may alleviate some of the above problems. In particular, the present invention aims to provide a straightforward method allowing to differentiate propagating cells of liver origin known as rat liver epithelial cell (RLEC) lines to hepatocyte-like cells. Thanks to the propagating nature of RLEC lines - up to 100 or more passages may be achieved *in vitro,* such cells could be routinely kept in the laboratory and differentiated when the need for hepatocyte-like cells arises.

To the inventors' best knowledge, the prior art does not teach a ready method, e.g., involving the addition of specific differentiation agents, to differentiate RLEC to hepatocyte-like cells.

Williams et al. 1971 (Exp Cell Res 69: 106-112), Guillouzo 1986 (in Research in Isolated and Cultured Hepatocytes, Guillouzo, A. and Guguen-Guillouzo, C., eds., John Libbey, London, UK, pp. 313-332) and Guguen-Guillouzo et al. 1983 (Exp Cell Res 143, 47-54) teach isolation of RLEC lines. Guguen-Guillouzo et al. 1983 teaches that RLEC in co-culture with primary hepatocytes help to maintain the differentiated properties of the latter.

WO 2006/045331 teaches differentiation of stem cells, in particular of multipotent adult progenitor cells (MAPC) and mesenchymal stem cells (MSC), into hepatocyte-like cells using sequential exposure of the said stem cells to certain differentiation agents. WO 2006/045331 does not specifically concern differentiation of RLEC cells, nor to use RLEC which are not normally considered stem cells.

The objects of the invention are addressed as described below.

### SUMMARY OF THE INVENTION

The present invention provides subject-matter as set forth in any one and all of (i) to (xv) below:
(i) A method for differentiating cells of a rat liver epithelial cell (RLEC) line to hepatocyte-like cells *in vitro,* comprising exposure of the cells of the RLEC line to two or more differentiation agents, wherein the cells are exposed sequentially to at least two of the said differentiation agents, and wherein two or more or all of the differentiation agents to which the cells of the RLEC line are exposed are chosen from fibroblast growth factor 4 (FGF-4), hepatocyte growth factor (HGF), insulin, transferrin, oncostatin M (OSM), or a biologically active variant or derivative of any of the above, selenium or a biologically acceptable acid or salt thereof, and a glucocorticoid.
(ii) The method as set forth in (i) above, comprising:
   (a) exposing the cells of the RLEC line to FGF-4 or a biologically active variant or derivative thereof during a time interval, the onset of which is between *t*=0 hours and *t*=72 hours, more preferably between *t*=0 hours and *t*=48 hours, even more preferably between *t*=0 hours and *t*=24 hours; and/or
   (b) exposing the cells of the RLEC line to HGF or a biologically active variant or derivative thereof during a time interval, the onset of which is between *t*=0 hours and *t*=96, preferably between *t*=0 hours and *t*=48 hours, and more preferably between *t*=0 hours and *t*=24 hours; and/or
   (c) exposing the cells of the RLEC line to insulin or a biologically active variant or derivative thereof during a time interval, the onset of which is between *t*=24 hours and *t*=240 hours, preferably between *t*=48 hours and *t*=192 hours, and more preferably between *t*=72 hours and *t*=144 hours; and/or
   (d) exposing the cells of the RLEC line to transferrin or a biologically active variant or derivative thereof during a time interval, the onset of which is between *t*=24 hours and *t*=240 hours, preferably between *t*=48 hours and *t*=192 hours, and more preferably between *t*=72 hours and *t*=144 hours; and/or
   (e) exposing the cells of the RLEC line to selenium or a biologically acceptable acid or salt thereof during a time interval, the onset of which is between *t*=24 hours and *t*=240 hours, preferably between *t*=48 hours and *t*=192 hours, and more preferably between *t*=72 hours and *t*=144 hours; and/or
   (f) exposing the cells of the RLEC line to a glucocorticoid during a time interval, the onset of which is between *t*=72 hours and *t*=240 hours, preferably between *t*=96 hours and *t*=192 hours; and/or
   (g) exposing the cells of the RLEC line to OSM or a biologically active variant or derivative thereof during a time interval the onset of which is between *t*=140 hours and *t*=480 hours, more preferably between *t*=264 hours and *t*=312 hours, even more preferably between *t*=276 hours and *t*=300 hours;
   wherein *t*=0 hours is the starting time point of the said method.
(iii) The method as set forth in (ii) above, comprising at least the step (b) and any one, any two or all three of steps (c), (d) and (e).
(iv) The method as set forth in (iii) above, additionally comprising one or both of the steps (f) and (g).
(v) The method as set forth in any of (i) to (iv) above, further comprising exposing the cells of the RLEC line to a histone deacetylase (HDAC) inhibitor.
(vi) The method as set forth in (v) above, wherein the cells are exposed to the HDAC inhibitor during a time interval, the onset of which is between *t*=0 hours and *t*=96 hours.
(vii) The method as set forth in (v) above, wherein the cells are exposed to the HDAC inhibitor during a time interval, the onset of which is between *t*=96 hours and *t*=480 hours, preferably at about *t*=144 hours or later.
(viii) The method as set forth in any of (v) to (vii) above, wherein said HDAC inhibitor is trichostatin A (TSA) or a salt thereof.
(ix) The method as set forth in any of (v) to (vii) above, wherein said HDAC inhibitor is chosen from compounds of formulas (I) to (XI) and salts thereof: 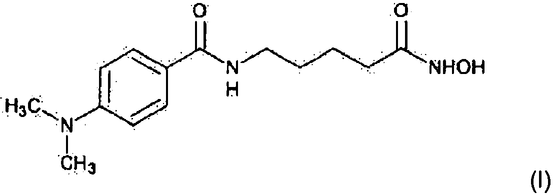 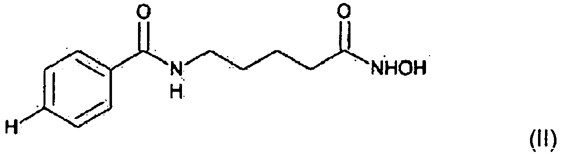 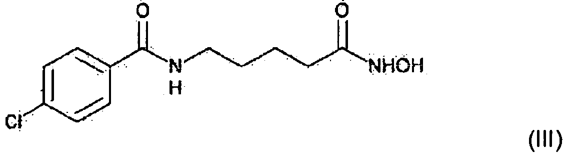 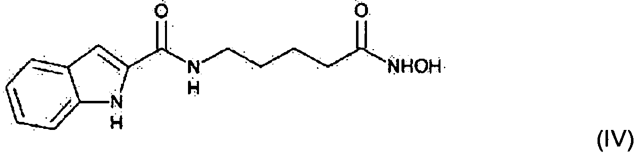 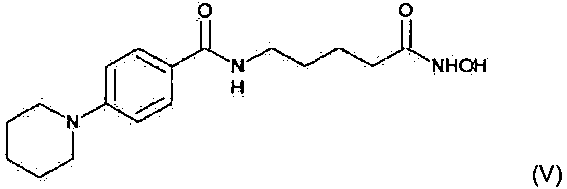 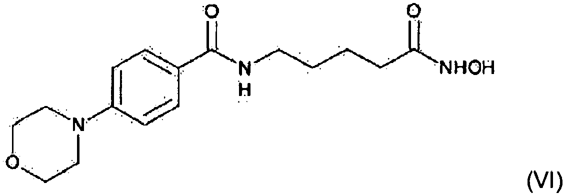 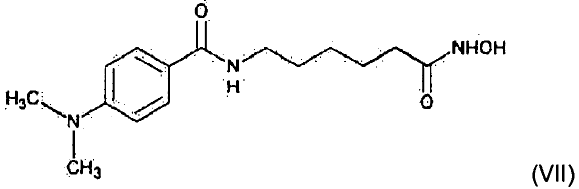 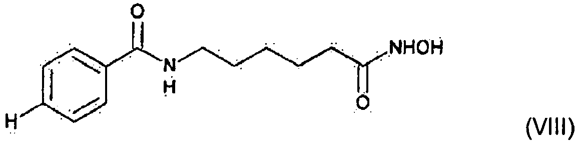 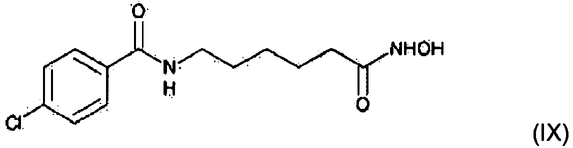 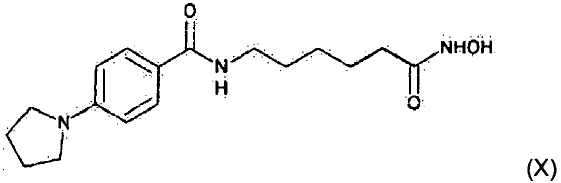 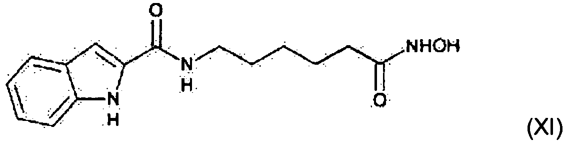
(x) The method as set forth in any one of (i) to (ix) above, comprising exposure of the cells of the RLEC line to the two or more differentiation agents, as follows:
   - days 0-1: FGF-4 or a biologically active variant or derivative thereof;
   - days 1-3: FGF-4 and HGF, or a biologically active variant or derivative thereof;
   - days 3-6: FGF-4, HGF, insulin and transferrin, or a biologically active variant or derivative thereof, and selenium or a biologically acceptable acid or salt thereof;
   - days 6-9: HGF, insulin and transferrin, or a biologically active variant or derivative thereof, selenium or a biologically acceptable acid or salt thereof, and a glucocorticoid;
   - days 9-12: HGF or a biologically active variant or derivative thereof and a glucocorticoid;
   - days 12-15: HGF and OSM, or a biologically active variant or derivative thereof, and a glucocorticoid;
   - from day 15 on: OSM or a biologically active variant or derivative thereof, and a glucocorticoid.
(xi) The method as set forth in any one of (i) to (ix) above, comprising exposure of the cells of the RLEC line to the two or more differentiation agents, as follows:
   - days 0-3: HGF or a biologically active variant or derivative thereof;
   - days 3-6: HGF, insulin and transferrin, or a biologically active variant or derivative thereof, and selenium or a biologically acceptable acid or salt thereof;
   - days 6-9: HGF, insulin and transferrin, or a biologically active variant or derivative thereof, selenium or a biologically acceptable acid or salt thereof, and a glucocorticoid;
   - days 9-12 HGF or a biologically active variant or derivative thereof and a glucocorticoid;
   - days 12-15: HGF and OSM, or a biologically active variant or derivative thereof, and a glucocorticoid;
   - from day 15 on: OSM or a biologically active variant or derivative thereof, and a glucocorticoid.
(xii) Use of rat hepatocyte-like cells or a cell population comprising such for the manufacture of a medicament for the treatment of liver diseases, wherein cells of RLEC line are exposed to two or more differentiation agents, wherein the cells of the RLEC line are exposed sequentially to at least two of the said differentiation agents, and wherein two or more or all of the differentiation agents to which the cells of the RLEC line are exposed are chosen from FGF-4, HGF, insulin, transferrin, OSM, or a biologically active variant or derivative of any of the above, selenium or a biologically acceptable acid or salt thereof, and a glucocorticoid, thereby differentiating the cells of the RLEC line to the hepatocyte-like cells *in vitro.*
(xiii) Method for assaying toxicity, carcinogenicity or biotransformation in hepatocyte-like cells or a cell population comprising such, or method for preparation of bio-artificial liver devices or liver assist devices comprising hepatocyte-like cells or a cell population comprising such, comprising the method as set forth in any one of (i) to (ix) above for differentiating cells of RLEC line to the hepatocyte-like cells *in vitro.*
(xiv) Method for providing a pharmaceutical composition comprising rat hepatocyte-like cells or a cell population comprising such, comprising the method as set forth in any one of (i) to (ix) above for differentiating cells of RLEC line to the hepatocyte-like cells *in vitro.*
(xv) The method as set forth in any one of (i), (ii), (x) or (xi) above, wherein the glucocorticoid is dexamethasone.

The subject-matter provided by the invention thus specifically belongs to the disclosure, description and teaching of the present specification.

The present specification describes an efficient method of obtaining hepatocyte-like cells from cells of liver epithelial cell (LEC) lines *in vitro,* comprising exposure of the cells of the LEC line to two or more differentiation agents, wherein the cells are exposed sequentially to at least two of the said differentiation agents.

While the method described herein may employ LEC cells from different species, including various mammalian species, and including man, i.e., LEC cells derived from human subjects, the present inventors particularly optimized the said method for cells of rat epithelial cell (RLEC) lines. In any instance, such cells may be advantageously obtained from readily available tissue and under proper culture conditions, may be stably kept in culture for a high number of passages, thereby providing for an ample cell source for the differentiation towards hepatocyte-like cells.

Preferably the present differentiation method as described herein may involve the sequential addition of specific differentiation agents, wherein at least some may be sequentially added. Such differentiation agents may be preferably chosen from the group comprising: fibroblast growth factor 4 (FGF-4), hepatocyte growth factor (HGF), insulin, transferrin, oncostatin M (OSM), or a biologically active variant or derivative of any of the above, selenium or a biologically acceptable acid or salt or derivative thereof, and a glucocorticoid, preferably dexamethasone.

In further embodiments, as detailed herein below the inventors optimized advantageous intervals during which the cells need to be exposed to each of the above differentiation agents, such as to achieve successful differentiation.

The present specification further describes hepatocyte-like cells obtainable using the present methods, compositions comprising such and specific uses thereof, such as in therapy, assays of toxicity, assays of carcinogenicity, assays of biotransformation, or in bio-artificial liver devices, liver assist devices. etc.

### BRIEF DESCRIPTION OF FIGURES

In all figures, time within the differentiation method is marked "D" (i.e., in days) and not "*t*" (i.e., in hours). Accordingly, "D0" refers to the start of differentiation, i.e., "*t*=0"; D0* denotes undifferentiated RLEC. The relationship between days (D) and hours (t) will be obvious to a skilled person.
**Figure 1** illustrates morphology of undifferentiated RLEC after 4 days (*1*) and 5 days (*2*) of expansion.
**Figure 2** illustrates characterisation of undifferentiated RLEC (D0*) by immunocytochemistry for CK18 (*1*), CK19 (*2*), c-kit (3), HNF-3beta (*4*), HNF-4 (*5*), HNF-1alpha (*6*), AFP (*7*), ALB (*8*), TTR (*9*), Cx43 (*10*), Cx32 (*11*), MRP2 (*12*), with (*a*) and without (*b*) DAPI counterstain. The shown data is representative for at least two experiments. Original magnification 32x10.
**Figure 3** illustrates morphology of differentiating RLEC subjected to the method as in example 5. Figure 3(A): morphology on *t*=6 days (*1*) and *t*=18 days (*2*-*3*) of the differentiation process. Arrows indicate bi-nucleated cells. The photographs are each representative of at least 5 experiments. Original magnification is 10x10 (*1*, *2*) and 40x10 (*3*). Figure 3(B): a further experiment showing morphology of RLEC differentiated according to example 5, optionally with TSA.
**Figure 4** illustrates expression of CK19 (*1*) and CK18 (*2*) by differentiating RLEC on *t*= 0, 6, 12 and 15 days or *t*=0, 6, 12 and 18 days, respectively, after initiation of the differentiation. (*a*) with and (*b*) without DAPI counterstain. Original magnification 32x10.
**Figure 5** illustrates expression of HNF-3beta (*1*), HNF-4 (*2*) and HNF-1alpha (*3*) by differentiating RLEC on the indicated days after initiation of the differentiation. (*a*) with and (*b*) without DAPI counterstain. Original magnification 32x10.
**Figure 6** illustrates expression of AFP (*1*), ALB (*2*) and TTR (*3*) by differentiating RLEC on the indicated days after initiation of the differentiation. (*a*) with and (*b*) without DAPI counterstain. Original magnification 32x10.
**Figure 7** illustrates expression of Cx32 (*1*), Cx43 (*2*) and MRP (*3*) by differentiating RLEC on the indicated days after initiation of the differentiation. (*a*) with and (*b*) without DAPI counterstain. Original magnification 6x32x10 (*1*, *2*) and 32x10 (*3*).
**Figure 8** shows effect of Jung-type HDACi on critical cell cycle mediators after 3 days of incubation in primary rat hepatocyte cultures. Assessed as in example 11 a.
**Figure 9** shows effect of Jung-type HDACi on the expression of phase I biotransformation markers CYP2 B1 (A) and CYP1 A1 (B), in primary rat hepatocyte cultures. Measured as in example 11 b.
**Figure 10** shows morphological appearance of primary rat hepatocytes cultured for 96h in differentiating promoting conditions under control conditions (untreated cells) (A) or in the presence of 10µM AN-6 (B), AN-8 (C) or AN-10 (D).
**Figure 11** illustrates HDAC inhibition potency of TSA (A), Jung type (B) and Jung-1 type (C) compounds as determined by the Fluor de Lys assay (example 9).
**Figure 12** shows dose-dependent inhibition of DNA synthesis by selected HDACi in proliferation-promoting culture of primary rat hepatocytes, measured as detailed in example 10a. Results are expressed as percentage of positive control (untreated cells cultured in the presence of mitogen). Total inhibition of hepatocyte proliferation is considered when DNA synthesis of treated cells equals DNA synthesis level of negative control (untreated cells cultivated in the absence of mitogen). Results shown are the means ± SD from 4 or more independent experiments.
**Figure 13** shows the effect of selected HDACi on the LDH leakage index in primary rat hepatocyte cultures, measured as described in example 10b. The results shown are the means ± SD from 3 independent experiments.
**Figure 14** illustrates the effect of TSA used alongside in the sequential differentiation protocol of the invention on the expression and activity of liver enriched transcription factors (LETFs) during hepatic differentiation of RLECs. A: HNF-3b, B: HNF-4, C: HNF-1a, a: with DAPI counterstain, b: without DAPI counterstain.
**Figure 15** illustrates morphology of RLEC differentiated in the presence of an HDAC inhibitor, as explained in example 13.

### DETAILED DESCRIPTION OF THE INVENTION

As used herein, the singular forms "a", "an", and "the" include both singular and plural referents unless the context clearly dictates otherwise. By way of example, "a cell" refers to one or more than one cell.

The terms "comprising", "comprises" and "comprised of" as used herein are synonymous with "including", "includes" or "containing", "contains", and are inclusive or open-ended and do not exclude additional, non-recited members, elements or method steps.

In an aspect, the invention relates to a method for differentiating cells of a rat liver epithelial cell (RLEC) line to hepatocyte-like cells *in vitro,* comprising sequential exposure of the cells of the RLEC line to at least two differentiation agents.

The term *"in vitro"* as used herein denotes outside, or external to, animal or human body. The term *"in vitro"* should be understood to encompass *"ex vivo",* which typically refers to tissues or cells removed from an animal or human body and maintained or propagated outside the body, e.g., in a culture vessel.

The terms "differentiation", "differentiating" or derivatives thereof as used herein denote a process by which an unspecialized or relatively less specialized cell becomes relatively more specialized. In the context of cell ontogeny, the adjective "differentiated" is a relative term. Hence, a "differentiated cell" is a cell that has progressed further down a certain developmental pathway than the cell it is being compared with. A relatively more specialized cell may differ from an unspecialized or relatively less specialized cell in one or more demonstrable phenotypic characteristics, such as, for example, the presence, absence or level of expression of particular cellular components or products, e.g., RNA, proteins or other substances, activity of certain biochemical pathways, morphological appearance, proliferation capacity and/or kinetics, differentiation potential and/or response to differentiation signals, etc., wherein such characteristics signify the progression of the differentiation towards the relatively more specialized cell.

In the present context, cells of an RLEC line are considered to be relatively less specialized and the method of the invention results in their differentiation towards hepatocyte-like cells, which are considered relatively more specialized by virtue of their displaying at least one and preferably more characteristics typical of parenchymal hepatocytes, as detalied eisewhere in this specification. Hence, as used herein, the term "differentiating" of RLEC to hepatocyte-like cells may be considered synonymous to the term "obtaining" hepatocyte-like cells from RLEC cells.

The method of the present invention uses cells of a rat liver epithelial cell (RLEC) line as a starting material for differentiation towards hepatocyte-like cells. However, a skilled person understands that the present or analogous method may be used for differentiation of LEC cells originating from other species, preferably various mammalian species, and specifically including LEC of human origin.

The term "RLEC" line is used in the art to denote any propagating epithelial cell line derived from neonatal rat liver according to methods established by Williams et al. 1971 (Exp Cell Res 69: 106-112), Guillouzo 1986 (in Research in Isolated and Cultured Hepatocytes, Guillouzo, A. and Guguen-Guillouzo, C., eds., John Libbey, London, UK, pp. 313-332) and Guguen-Guillouzo et al. 1983 (Exp Cell Res 143, 47-54). Without being limited to any hypothesis, it has been suggested in the art that RLEC or progenitors thereof may originate from the non-parenchymal compartment of the rat liver organ, not excluding other species, and in particular from the biliary epithelium.

In general, the above methods for preparation of RLEC lines comprise the following key steps: 1) liver fragments are obtained from livers of neonatal rats, such as 10-day old rats, usually by fine chopping of the liver organ; 2) the said liver fragments are incubated in a buffer solution comprising trypsin to disassociate the tissue into cells, such as in a PBS or HEPES-buffered solution of 0.25% trypsin for 15 minutes; 3) usually, the so obtained cell composition may be washed one or more times with an isotonic buffer, such as with PBSA; 4) fibroblastic cells are substantially eliminated from the said cell composition by means of their faster attachment to tissue culture plastic, as follows: the cells are plated onto a tissue culture plastic substrate, such as a tissue culture vessel (culture vessel No. 1), and allowed to attach for 20 minutes, the supernatant containing cells which have not attached to the substrate plastic is then collected and re-plated onto a fresh tissue culture vessel (culture vessel No. 2), the above steps of 20-minute attachment and re-plating of supernatant onto a fresh culture vessel are repeated at least two more times, thereby obtaining at least culture vessels Nos. 3 and 4 which may mainly contain epithelial cells; 5) the said epithelial cells from these culture vessels are subsequently cultured in Williams' medium E supplemented with between 5 and 10% fetal bovine serum, to obtain an population (RLEC iine), which is then maintained by serial passages in the said medium.

Accordingly, in an embodiment, an RLEC line as used herein denotes a cell population obtainable according to any of the above referenced methods.

In a preferred embodiment, an RLEC line suitable for use in the present invention may denote a cell population obtainable according to the method detailed in example 1 and in Henkens et al. 2006 ("Rat hepatocyte cultures: conventional monolayer cultures and cocultures with rat liver epithelial cells, In: Cytochrome P450 Protocols: Second Edition. Series in: Methods in Molecular Biology, Phillips I.R. and Shephard E.A., eds., vol. 320, pp. 239-254, Humana Press Inc., Totowa, NJ).

A skilled person will appreciate that other methods may be employed to obtain liver epithelial cell lines from other organisms, such as from mammals, and especially from human tissues.

It shall be understood that the term RLEC line also encompasses the progeny of an RLEC cell population obtainable as above, or the progeny of a fraction of the said cell population. For example, such progeny may be a non-clonal line, i.e., containing the offspring of multiple cells or cells from multiple colonies of an RLEC cell population obtainable as above; or such progeny may be a clonal sub-line, i.e., derived from a single cell or a single colony of the RLEC cell population.

It shall further be understood that the term RLEC line also encompasses the above cells or progeny thereof that have been stably or transiently transfected or transformed with a nucleic acid of interest, e.g., prior to use in the method of the invention. Nucleic acid sequences of interest may include, but are not limited to, e.g., those encoding gene products which may enhance the survival, differentiation and/or functional aspects of hepatocyte-like cells, or to deliver a therapeutic gene to a site of administration of such cells. For example, the above genetic modification may result in upregulation or downregulation of the expression of one or more genes that are normally expressed in RLEC cells, and/or in hepatocyte-like cells obtainable therefrom, and/or in parenchymal hepatocytes, or may introduce the expression of a gene that is normally not expressed in the RLEC cells, and/or hepatocyte-like cells obtainable therefrom, and/or in parenchymal hepatocytes; such gene may be native or heterologous to the cells. A vector is designed using the known encoding sequence for the desired gene, operatively linked to a promoter that is either pan-specific or specifically active in the RLEC line and/or the differentiated hepatocyte-like cells. The said genetic modification may be carried out at the level of the cells of an RLEC iine, or may be carried out on the rat subject or ancestors thereof, from which the RLEC line is obtained.

A skilled person further understands that isolation and maintenance of RLEC lines requires adherence to correct cell culture practice and that RLEC lines when appropriately cultivated can be kept for many passages, e.g., for up to 100 passages or more, while maintaining stable phenotypic characteristics. Conversely, suboptimal culture practice or other influences, e.g., the effect of different FBS lots or batches, may cause the RLEC lines to undergo spontaneous transformation, as evidenced by, e.g., (gradual) loss of cell contact inhibition at confluence and a change of adhesion properties (esp. increased sensitivity to calcium). A skilled person is able to recognize such phenotypic alterations of RLEC lines and to select those RLEC lines which display the desired phenotype for use in the present method.

In further embodiments, an RLEC line suitable for use in the present invention, preferably derived as above, may display at least one, and preferably at least two or more, e.g., two, three, four, five or more, or all, of the following: the presence of the expression of cytokeratin 18 (CK18), the presence of the expression of cytokeratin 8 (CK8), the presence of the expression of cytokeratin 14 (CK14), the presence of the expression of cytokeratin 19 (CK19), the presence of the expression of connexin 43, the presence of the expression of at least one phase I biotransformation enzyme (e.g., of CYP2E1 and/or epoxidehydrolase), the presence of the expression of at least one phase II biotransformation enzyme (e.g., of ST and/or glutathione-S-transferase), the presence of the expression of HNF3beta but typically the absence of the activity thereof, the presence of expression of HNF1 and typically also activity thereof, the presence of the expression of transthyretin (pre-albumin), the presence of the expression of MRP2, the secretion of fibronectin, the secretion of collagen type I and/or III, the absence of the expression of alpha-fetoprotein (AFP), the absence of the expression of albumin (ALB), the absence of the expression of connexin 32, cKit, HNF4, and contact inhibition of growth upon reaching confluence.

The presence or absence of the expression and/or secretion of the above proteins can be detected using any suitable immunological technique known in the art, such as flow cytometry, immuno-cytochemistry or affinity adsorption, Western blot analysis, ELISA, etc., or by any suitable technique of measuring the quantity of the corresponding mRNA, e.g., Northern blot, semi-quantitative or quantitative RT-PCR, etc. In addition, the presence of the expression of enzymes may be detected by an assay for their respective enzymatic activity. Wherein a cell is said to express a particular protein, this means that a skilled person will conclude the presence or evidence of a distinct signal, e.g., antibody-detectable, for that protein when carrying out the appropriate measurement, compared to a suitable control.

In a preferred embodiment, an RLEC line suitable for use in the present invention, preferably derived as above, may display at least one, and preferably at least two, three or all of the following: the presence of the expression of at least one of CK8 or CK18 and at least one of CK19 or CK14; the presence of expression of connexin 43 and the absence of the expression of connexin 32; the absence of the expression of AFP and ALB; contact inhibition of growth upon reaching confluence.

In a preferred embodiment, an RLEC line suitable for use in the present invention, preferably derived as above, preferably having characteristics as defined above, is capable of supporting long-term (e.g., 2 weeks or more, e.g., 4 weeks or more, e.g., up to 10 weeks or more) survival of primary rat hepatocytes in a co-culture in vitro, when used as described by Guguen-Guillouzo et al. 1983. Specifically, in preferred embodiments, the functionality of the rat hepatocytes in co-culture may be as described in any or more of: Akrawi et al. 1993 (Biochem Pharmacol. 1993 Apr 22;45(8):1583-91), Coecke et al. 1998 (Biochem. Pharmacol. 56: 1047-1051), Coecke et al. 2000 (J Endocrinol. 166(2):363-71), Rogiers et al. 1990 (Biochem Pharmacol. 40(8):1701-6, Rogiers et al. 1992 (Arch Toxicol Suppl. 15:156-9), Vandenberghe et al. 1989 (FEBS Lett 251(1-2):59-64), Vandenberghe et al. 1990 (Mol Pharmacol 37(3):372-6. Erratum in: Mol Pharmacol 1990;38(5):751), Vandenberghe et al.

As explained above, an RLEC line according to the invention can be defined by its isolation from rat liver using specific protocols. A skilled person shall understand that the above methods may be altered, or other protocols may be established, which would still yield cells substantially identical to RLEC lines as obtainable by the above methods. Therefore, such cells obtained by such altered or further protocols would fall within the term RLEC line as intended herein.

Analogously, the above methods employ neonatal rat livers for isolation of RLEC lines. However, a skilled person shall understand that if cells substantially identical to RLEC lines as obtainable by the above methods could be obtained from livers at a different stage, e.g., prenatal or adult stage, such cells would also fall within the term RLEC line as intended herein.

The method of the present invention differentiates cells of an RLEC line towards hepatocyte-like cells. As used herein, the term "hepatocyte-like cell" refers to a cell which, relative to cells of an RLEC line, displays at least one and preferably more properties that are characteristic of mature, parenchymal hepatocytes. The above expression "relative to" means that hepatocyte-like cells may display a property which has not been present in cells of the RLEC cell line prior to being subjected to the method of the invention, or which has been quantitatively less present in the said cells of the RLEC cell line.

Preferably, a hepatocyte-like cell may display at least one and preferably at least two, three, four, five or more of the following properties: ability to use pyruvate as a sole carbon source; phase I biotransformation capacity (e.g. ethoxyresorufin, pentoxyresorufin, testosterone); phase II biotransformation capacity (e.g. 1-chloro-2,4 dinitrobenzene, 1,2-dichloro-4-nitrobenzene, 7-chloro-4-nitrobenzene-2-oxa-1,3-diazole, estradiol, estrogen), the presence of cytochrome P450 protein and gene expression; inducibility of phase I and phase II biotransformation enzymes (e.g. beta-napthoflavone , phenobarbital, methylcholanthrene); albumin secretion, urea production, glycogen storage, the presence of the expression of one or more of ALB, AFP, gamma-glutyryltransferase, hepatocyte nuclear factor (HNF) 1alpha, HNF 1beta, HNF 3alpha, HNF 3beta, HNF 4, HNF-6, anti-trypsin, CX32, MRP2, C/EBPalfa, transthyretin, CK-18 and/or CFTR; polygonal morphology; the presence of more than 1 nucleus in a cell, preferably 2 nuclei in a cell.

A skilled person appreciates, e.g., by assessing one or more of the above properties, that an RLEC cell subjected to the present method undergoes a change towards an increasingly hepatocyte-like phenotype. He also understands that for a hepatocyte-like cell of the invention to be useful in various applications, such cell need not be identical to, e.g., need not have all properties of a mature, parenchymal hepatocyte. By means of example and not limitation, expression of appropriate biotransformation enzymes in such cells may be employed to assess the metabolism of drugs, regardless of whether the cells may display other hepatocyte properties.

In the present method, differentiation of cells of an RLEC line to hepatocyte-like cells *in vitro* comprises sequential exposure of the said cells to at least two differentiation agents.

The terms "sequential" or "sequentially" describe exposing cells to two or more differentiation agents, wherein the cells are exposed to each differentiation agent during a respective time interval, and wherein the onset of the respective time interval, i.e., the time point at which the exposure of the cells to the corresponding differentiation agent begins, differs for at ieast two of the said differentiation agents. Hence, in the present method, the cells are exposed to at least one differentiation agent starting at an earlier time point than to one or more other differentiation agents. However, this does not exclude that the onset of exposure may be the same for at least some of the differentiation agents. In other words, in the present method, differentiation of cells of an RLEC line to hepatocyte-like cells *in vitro* comprises exposure to two or more differentiation agents, wherein the cells are exposed sequentially to at least two of the said differentiation agents.

Usually, in the instant method, the respective time interval during which the cells are exposed to a given differentiation agent is continuous, i.e., the cells are exposed to the differentiation agent throughout the length of the said time interval. The concentration of the differentiation agent to which the cells are exposed during the said time interval may be altered, e.g., to elicit a varying magnitude of biological response in the cells. For example, in embodiments the concentration of a given differentiation agent may be substantially constant throughout the time interval of exposing the cells thereto, or the concentration may, within the said time interval, have a trend which is ascending, constant, descending, or the combination thereof.

Further, the respective time interval during which the cells are exposed to a given differentiation agent may have a discrete endpoint, i.e., the time point at which the exposure of the cells to the said differentiation agent, or at least to a biologically effective quantity thereof (i.e., a quantity which elicits a desired biological response in so treated cells), is discontinued. Otherwise, such time interval may have an open end, i.e., the exposure to the said differentiation agent can be continued essentially for as long as the cells subjected to the present method are kept in cell culture. For example, continuous exposure to one or more differentiation agents may be required to maintain at least some of the differentiated properties of the cells.

The respective time intervals during which the cells are exposed to the different differentiation agents may be discrete from each other, or such time intervals for at least some of said differentiation agents may partly or wholly overlap. In the latter instance, the cells would thus be exposed to a combination of the said differentiation agents during the period of the overlap.

In the present method, cells are thus differentiated *in vitro* through exposure to two or more differentiation agents, at least two of which are contacted with the cells sequentially, as opposed to being administered at the same time point.

The term "differentiation agent" as used herein generally encompasses any agent, such as, e.g., a chemical and/or biological substance or composition, which exerts a desirable effect on the differentiation and/or maturation of said cells. In embodiments, a differentiation agent may be a growth factor, a cytokine, an interleukin, a hormone, a protein, an enzyme, a vitamin, a metabolite, a nutrient, a small molecule, a chemical compound, a solvent, or a chemical element.

In an embodiment, the cells are sequentially exposed to two or more differentiation agents which, individually and/or in cooperation, promote differentiation towards and/or maturation of a hepatic phenotype.

In a preferred embodiment, the cells are sequentially exposed to two or more differentiation agents, wherein at least one and preferably at least two or more, e.g., three, four, five, six, seven or all of the said differentiation agents are chosen from the group comprising: fibroblast growth factor 4 (FGF-4), hepatocyte growth factor (HGF), insulin, transferrin, oncostatin M (OSM), or a biologically active variant or derivative of any of the above, selenium or a biologically acceptable acid or salt or derivative thereof, and a glucocorticoid.

The above proteinaceous substances, in particular the growth factors FGF-4, HGF, insulin, transferrin and oncostatin M, are well-characterised as such, including primary amino acid sequence of their precursors and their mature forms from various animal, and particularly mammalian species, their processing, isoforms, activity, binding to cognate receptors, etc. A skilled person can readily access information and annotation on these molecules via publicly accessible databases, such as GenBank (www.ncbi.nih.gov) and Swissprot (www.expasy.org). By means of guidance and not limitation, Swissprot accession numbers for several of these polypeptides and/or their precursors are listed here: FGF-4: P08620 (human), P11403 (mouse), P48804 (chicken), P48803 (bovine); HGF: P14210 (human), Q08048 (mouse), P17945 (rat); insulin: P01308 (human), P01317 (bovine), P01321 (dog), P67970 (chicken), P01310 (horse), P01315 (pig), P21563 (rodent species); transferrin: P02787 (human), Q921I1 (mouse), P09571 (pig), P12346 (rat), Q29443 (bovine); OSM: P13725 (human), P53347 (mouse), P53346 (bovine).

Where the present method employs a differentiation agent which is a peptide, polypeptide or a proteinaceous entity, such as a growth factor, and in particular FGF-4, HGF, insulin, transferrin and/or OSM, the said differentiation agent may be substantially the same as the corresponding, naturally occurring substance, e.g., a naturally occurring growth factor. For example, the primary amino acid sequence of the constituent peptide(s) or polypeptide(s) of the said differentiation agent may comprise or consist of a sequence identical to that of the corresponding, naturally occurring growth factor (e.g., may be the same as in the above listed Swissprot accession numbers). Also, the secondary and potentially higher order structure of the said constituent peptide(s) or polypeptide(s) of the differentiation agent, the post-translational modification(s) thereof, as well as the covalent (e.g., disulfide bonds) and/or noncovalent interactions there between, may be substantially the same for the said differentiation agent as in the corresponding, naturally occurring growth factor. Generally as a consequence of such structural similarity, the biological effects of the differentiation agent would be qualitatively and possibly also quantitatively comparable to that of the corresponding, naturally occurring growth factor.

The term "naturally occurring" is used to describe an entity that can be found in nature as distinct from being artificially produced by man. For example, a polypeptide sequence present in an organism, which can be isolated from a source in nature and which has not been intentionally modified by man in the laboratory, is naturally occurring. When referring to a particular entity, e.g., to a peptide, polypeptide or a protein entity, and in particular FGF-4, HGF, insulin, transferrin and/or OSM, the term encompasses all forms and variants thereof which may occur in nature, e.g., due to normal allelic variation between individuals of a species, due to differences of transcription (e.g., due to the use of alternative promoters), post-transcriptional modifications (e.g., due to differences of mRNA splicing, mRNA editing, etc.), and/or of post-translational modifications (e.g., due to differences of proteolytic processing, disulfide bonds, amino acid modifications such as glycosylation, phosphorylation, etc.).

Moreover, the differentiation agent to be used in the present method, in particular FGF-4, HGF, insulin, transferrin and/or OSM, may be substantially the same as the corresponding, naturally occurring substance of an animal species, preferably of a mammalian species, such as, e.g., human, mice, rat, rabbit, dog, cat, cow, horse, pig or primate, e.g., monkey and ape, or the like. A skilled person will be able to determine whether a particular differentiation agent derived from a certain animal or mammalian species elicits a desired biological effect when applied to the cells in the present method.

The present method may further employ, as differentiation agents, biologically active variants or derivatives of naturally occurring substances, e.g., of growth factors, in particular of FGF-4, HGF, insulin, transferrin and/or OSM. In the method of the invention, "biologically active" variants or derivatives of achieve at least about the same degree of differentiation of the treated cells towards hepatocytic phenotype as the respective naturally occurring substance or growth factor, when other conditions are substantially the same.

Where a growth factor exerts its effects by binding to its cognate receptor, biologically active variants or derivatives of the said growth factor may display affinity and/or specificity for binding to that cognate receptor, which is at least about as high as the affinity and/or specificity of the growth factor for binding thereto. For example, the said biologically active variants or derivatives may have affinity and/or specificity for binding to the cognate receptor which is at least 80%, e.g., at least 85%, preferably at least 90%, e.g., at least 90%, or even 100% or more of the affinity and/or specificity of the respective growth factor for binding to that receptor. The above parameters of the binding may be readily determined by a skilled person using *in vitro* or cellular assays which are known *per se.*

Where the activity of a given growth factor can be readily measured in an established assay, e.g., an *in vitro* or cellular assay (such as, for example, measurement of mitogenic activity in cell culture), biologically active variants or derivatives of the said growth factor may display activity in such assays, which is at least about as high as the activity of the growth factor. For example, the said biologically active variants or derivatives may show activity which is at least 80%, e.g., at least 85%, preferably at least 90%, e.g., at least 90%, or even 100% or more of the activity of the respective growth factor.

A "variant" of a polypeptide has an amino acid sequence which is substantially identical (i.e., largely but not wholly identical) to the amino acid sequence of the polypeptide. Herein, "substantially identical" refers to at least 85% identical, e.g., at least 90% identical, preferably at least 95% identical, e.g., least 99% identical. Sequence differences may result from insertion (addition), deletion and/or substitution of one of more amino acids.

Sequence identity between two polypeptides can be determined by aligning the amino acid sequences of the polypeptides and scoring, on one hand, the number of positions in the alignment at which the polypeptides contain the same amino acid residue and, on the other hand, the number of positions in the alignment at which the two polypeptides differ in their sequence. The two polypeptides differ in their sequence at a given position in the alignment when the polypeptides contain different amino acid residues at that position (amino acid substitution), or when one of the polypeptides contains an amino acid residue at that position while the other one does not or *vice versa* (amino acid insertion/addition or deletion). Sequence identity is calculated as the proportion (percentage) of positions in the alignment at which the polypeptides contain the same amino acid residue versus the total number of positions in the alignment.

At least some of the differences between the amino acid sequences of a variant and of the respective polypeptide with which the variant is substantially identical, can involve amino acid substitutions. Preferably, at least 85%, e.g., at least 90%, more preferably at least 95%, e.g., 100% of the said differences can be amino acid substitutions. Preferably, the said amino acid substitutions may be conservative. The term "conservative substitution" as used herein denotes that one amino acid residue has been replaced by another, biologically similar amino acid residue. Non-limiting examples of conservative substitutions include the substitution of one hydrophobic amino acid residue, such as isoleucine, valine, leucine or methionine for another, or the substitution of one polar residue for another, such as between arginine and lysine, between glutamic and aspartic acids or between glutamine and asparagine, and the like.

The term variant of a polypeptide also encompasses fragments of that polypeptide.

A variant growth factor may be comprised of one or more peptide(s) or polypeptide(s), at least one of which is a variant as defined above of the respective constituent peptide or polypeptide of the corresponding growth factor.

A "derivative" of a polypeptide is derivatised by chemical alteration of one or more amino acid residues and/or addition of one or more moieties at one or more amino acid residues, by glycosylation, phosphorylation, acylation, acetylation, sulphation, lipidation, a alkylation Typically, less than 50%, e.g., less than 40%, preferably less than 30%, e.g., less than 20%, more preferably less than 15%, e.g., less than 10% or less than 5%, e.g., less than 4%, 3%, 2% or 1% of amino acids in a derivative polypeptide may be so derivatised. A derivative proteinaceous growth factor may be comprised of one or more peptide(s) or polypeptide(s), at least one of which may be derivatised on at least one amino acid residue.

In an embodiment, the differentiation agent used in the present method, such as a growth factor, and in particular FGF-4, HGF, insulin, transferrin and/or OSM, may be isolated from an organism in which this naturally occurs.

In a preferred embodiment, the differentiation agent used in the method, such as a growth factor or a biologically active variant or derivative thereof is recombinant, i.e., produced by a host organism through the expression of a recombinant nucleic acid molecule, which has been introduced into the host organism or an ancestor thereof, and which comprises a sequence encoding the said polypeptide. The term "recombinant nucleic acid molecule" as used herein refers to a nucleic acid molecule (e.g., a DNA or cDNA molecule) which is comprised of segments joined together using recombinant DNA technology. The use of recombinantly expressed growth factors or biologically active variants or derivatives thereof may be particularly advantageous. For example, a recombinant growth factor may be more readily prepared from a recombinant source than by isolation from biological material.

Suitable expression systems, e.g., expression vectors, such as plasmid and viral vectors; host organisms, such as bacteria (e.g., *E. coli, S. tymphimurium, Serratia* marcescens, *Bacillus subtilis),* yeast (e.g., *S. cerevisiae* and *Pichia pastoris*), cultured plant cells (e.g., from *Arabidopsis thaliana* and *Nicotiana tobaccum*) and animal cells (e.g., mammalian cells and insect cells), and multi-cellular organisms, such as plants or animals; and procedures for isolation of the expressed recombinantly produced proteins, such as growth factors or biologically active variants or derivatives thereof, are known in the art. Reference is made to well-known textbooks, including, e.g., "Molecular Cloning: A Laboratory Manual, 2nd Ed." (Sambrook et al., 1989), Animal Cell Culture (R. I. Freshney, ed., 1987), the series Methods in Enzymology (Academic Press), Gene Transfer Vectors for Mammalian Cells (J. M. Miller & M. P. Calos, eds., 1987); "Current Protocols in Molecular Biology and Short Protocols in Molecular Biology, 3rd Ed." (F. M. Ausubel et al., eds., 1987 & 1995); Recombinant DNA Methodology II (R. Wu ed., Academic Press 1995), incorporated by reference herein. Recombinant growth factors are also commonly commercially available (e.g., from Sigma, Biological Industries, R&D Systems, Peprotech, etc.).

As mentioned, a differentiation agent may also be a glucocorticoid, such as, e.g., one chosen from the group consisting of dexamethasone, hydrocortisone, prednisolone, methylprednisolone, prednisone, triamcinolone, corticosterone, fluocinolone, cortisone, betamethasone, and preferably dexamethasone, or derivatives of any of the above, e.g., derivatives with better efficiency, stability, release, etc. By means of example and not limitation, cyclodextrin complexes, e.g., of dexamethasone, may be used.

An exemplary but non-limiting selenium comprising substance for use in the invention is sodium selenite. Other selenium salts or derivatives, e.g., with similar effect as the above may be used.

In the present method, exposing cells to a differentiation agent means that the cells are, either directly or indirectly, contacted with or brought together with the said differentiation agent, thereby facilitating interactions there between. Typically, cells may be exposed to a differentiation agent through the inclusion of the latter in the liquid media, in which the cells are cultured.

A differentiation agent can be included in a medium at a concentration sufficient to elicit a biological response to the said differentiation agent from cells contacted with the said medium. Biologically effective concentrations of differentiation agents useful in the present invention are generally known in the art and can be readily tested by a skilled person. By way of guidance and not limitation, concentrations at which particular differentiation agents may be used in the present method are given here below.

A proteinaceous growth factor, in particular FGF-4, HGF and oncostatin M, or a biologically active variant or derivative of any of the above, may be typically included in a medium at a final concentration of between 0.01ng/ml and 10µg/ml, preferably between 0.1ng/ml and 1µg/ml or between 0.5ng/ml and 500ng/ml, more preferably between 1ng/ml and 100ng/ml, e.g., between 1 and 50ng/ml, such as at about 5ng/ml, about 10ng/ml, about 20ng/ml, about 30ng/ml, about 40ng/ml or about 50ng/ml; insulin or a biologically active variant or derivative thereof can be typically used at concentrations between about 0.1µg/l and 1mg/l and preferably between about 1µg/l and 100µg/l, e.g., at about 25-50µg/l; transferrin or a biologically active variant or derivative thereof can be typically used at concentrations between about 0.1µg/l and 1mg/l and preferably between about 1µg/ml and 100µg/ml, e.g., at between about 25 and 55µg/l; selenium can be typically used at concentrations between about 0.1ng/l and 1µg/l, preferably between 1ng/l and 100ng/l, such as between 1ng/l and 50ng/l, preferably between about 25ng/l and 50ng/l; glucocorticoid, esp. dexamethasone can be typically used at concentrations between about 0.1nM and 1µM, preferably between about 1nM and 100nM, e.g., at about 10nM, about 20nM, about 30nM, about 40nM, or about 50nM.

In an embodiment, the cells are sequentially exposed to two or more differentiation agents, wherein at least one of the said differentiation agents is HGF or a biologically active variant or derivative thereof.

Preferably, when the starting time point of the present method for differentiation of cells (i.e., the time point at which the medium in which the cells are normally propagated or cultured without differentiation is supplemented with at least one differentiation agent according to the invention or is substituted with a medium comprising the said at least one differentiation agent) is denoted t=0 hours, the cells may be exposed to HGF or a biologically active variant or derivative thereof during a time interval, the onset of which is between *t*=0 hours and t=96 hours, e.g., at about *t*=0 hours, *t*=24 hours, *t*=48 hours, *t*=72 hours or *t*=96 hours, preferably between *t*=0 hours and *t*=48 hours, more preferably between *t*=0 hours and *t*=24 hours, e.g., at about *t*=0 hours, *t*=6 hours, *t*=12 hours, *t*=18 hours or *t*=24 hours. Hence, the cells begin to be exposed to HGF or a biologically active variant or derivative thereof at the above specified onset times within the present method.

In an embodiment, exposure of the cells to HGF or a biologically active variant or derivative thereof may continue for as long as the cells obtained by the differentiation method are maintained in culture. In other embodiments, the time interval during which the cells are exposed to HGF or a biologically active variant or derivative thereof may have a discrete endpoint, such as an endpoint between *t*=144 hours and *t*=480 hours, preferably between *t*=240 hours and *t*=480 hours, even more preferably between *t*=288 hours and *t*=360 hours, e.g., at about *t*=288 hours, *t*=312 hours, *t*=336 hours or *t*=360 hours.

In further embodiments, the cells are sequentially exposed to two or more differentiation agents, wherein at least one of the said differentiation agents is insulin or a biologically active variant or derivative thereof, and/or is transferrin or a biologically active variant or derivative thereof, and/or is selenium or a biologically acceptable acid or salt or derivative thereof.

Preferably, the cells may be exposed to any one or more of these agents during a time interval the onset of which is, independently for each of these agents, between *t*=24 hours and *t*=240 hours, preferably between *t*=48 hours and *t*=192 hours, and more preferably between *t=72* hours and *t*=144 hours, such as, e.g., at about *t=72* hours, *t*=96 hours, *t*=120 hours, or *t*=144 hours.

In an embodiment, exposure of the cells to insulin or a biologically active variant or derivative thereof, and/or transferrin or a biologically active variant or derivative thereof, and/or selenium or a biologically acceptable acid or salt or derivative thereof may, independently for each of these agents, continue for as long as the cell obtained by the differentiation method are maintained in culture. Alternatively, the said exposure may have, independentiy for each of these agents, a discrete endpoint, such as an endpoint between *t*=144 hours and *t*=480 hours, preferably between *t*=216 hours and *t*=360 hours, even more preferably between *t*=216 hours and *t*=288 hours, e.g., at about *t*=216 hours, *t*=240 hours, *t*=264 hours or *t*=288 hours.

In a particular embodiment, cells may be simultaneously exposed to any two or all three of insulin or a biologically active variant or derivative thereof, transferrin or a biologically active variant or derivative thereof, and selenium or a biologically acceptable acid or salt or derivative thereof, i.e., the respective time intervals of exposure to these agents may have the same onset and endpoints, as specified above.

In a further embodiment, the cells are sequentially exposed to two or more differentiation agents, wherein at least one of the said differentiation agents is a glucocorticoid, preferably dexamethasone. The cells may be exposed to the said glucocorticoid during a time interval the onset of which is preferably between *t*=72 hours and *t*=240 hours, more preferably between *t*=96 hours and *t*=192 hours, e.g., at about *t*=96 hours, *t*=120 hours, *t*=144 hours, *t*=168 hours, or *t*=192 hours, and most preferably at about *t*=144 hours. In a preferred embodiment, exposure of the cells to the glucocorticoid may continue for as long as the cells obtained by the differentiation method are maintained in culture.

In another embodiment, the cells are sequentially exposed to two or more differentiation agents, wherein at least one of the said differentiation agents is OSM or a biologically active variant or derivative thereof. The cells may be exposed to the said OSM or a biologically active variant or derivative thereof during a time interval the onset of which is preferably between *t*=140 hours and *t*=480 hours, more preferably between *t*=264 hours and *t*=312 hours, even more preferably between *t*=276 hours and *t*=300 hours, and most preferably at about *t*=288 hours. In a preferred embodiment, exposure of the cells to the OSM or a biologically active variant or derivative thereof may continue for as long as the cells obtained by the differentiation method are maintained in culture.

In yet another embodiment, the cells are sequentially exposed to two or more differentiation agents, wherein at least one of the said differentiation agents is FGF-4 or a biologically active variant or derivative thereof. The cells may be exposed to the said FGF-4 or a biologically active variant or derivative thereof during a time interval the onset of which is preferably between *t*=0 hours and *t*=72 hours, more preferably between *t=0* hours and *t*=48 hours, even more preferably between *t*=0 hours and *t*=24 hours, and most preferably at *t*=0 hours. In a preferred embodiment, the said exposure has a discrete endpoint, such as an endpoint between *t*=24 hours and *t*=144 hours, e.g., at about *t*=24 hours, *t*=48 hours, *t*=72 hours, *t*=96 hours, *t*=120 hours, or *t*=144 hours.

In various embodiments, the present invention envisages methods wherein the cells are sequentially exposed to two or more differentiation agents, comprising or consisting of exposure of the cells to two or more, e.g., two, three, four, five, six or all seven of the above specified differentiation agents, preferably at the above specified time intervals of exposure for each particular differentiation agent.

By means of example, in an embodiment the method may comprise exposure of the cells to HGF or a biologically active variant or derivative thereof and exposure to any one, two or preferably all of insulin or a biologically active variant or derivative thereof, transferrin or a biologically active variant or derivative thereof, and selenium or a biologically acceptable acid or salt or derivative thereof, preferably at the above specified time intervals of exposure to each respective differentiation agent.

This and other exemplary but non-limiting embodiments of the method (denoted A to M) are schematically illustrated in the following Table:

| Exposure to | A | B | C | D | E | F | G | H | I | J | K | L | M |
|---|---|---|---|---|---|---|---|---|---|---|---|---|---|
| FGF-4 or b.a. variant or derivative thereof | - | + | - | + | - | - | + | + | +/- | +/- | +/- | +/- | +/- |
| HGF or or b.a. variant or derivative thereof | + | + | + | + | + | + | + | + | + | + | + | + | + |
| Insulin or or b.a. variant or derivative thereof | + | + | + | + | + | + | + | + | + | + | - | - | - |
| Transferrin or or b.a. variant or derivative thereof | + | + | + | + | + | + | + | + | - | + | + | + | - |
| Selenium or a b.a. acid or salt or derivative thereof | + | + | + | + | + | + | + | + | - | - | - | + | + |
| Glucocorticoid, esp. dex | - | - | + | + | - | + | - | + | +/- | +/- | +/- | +/- | +/- |
| OSM | - | - | - | - | + | + | + | + | +/- | +/- | +/- | +/- | +/- |

"+": cells exposed to the differentiation agent; "-": cells not exposed to the differentiation agent; "+/-": cells exposed or not exposed, respectively; to the differentiation agent Further exemplary but non-limiting embodiments of the method related to the above embodiments A to M, also with indication of time intervals (columns, in days, 1 day=24 hours) of exposure to each differentiation agent are listed in the following tables:

### Embodiment E1:

| | 0 | 1 | 2 | 3 | 4 | 5 | 6 | 7 | 8 | 9 | 1 0 | 1 1 | 1 2 | 1 3 | 1 4 | 1 5 | 1 6 | 1 7 | 1 8 | 1 9 | 20 etc. |
|---|---|---|---|---|---|---|---|---|---|---|---|---|---|---|---|---|---|---|---|---|---|
| HGF or or b.a. variant or derivative thereof | + | + | + | + | + | + | + | + | + | + | + | + | + | + | + | + | + | + | + | + | + |
| Insulin or or b.a. variant or derivative thereof | - | - | - | - | - | - | + | + | + | + | + | + | + | + | + | + | + | + | + | + | + |
| Transferrin or or b.a. variant or derivative thereof | - | - | - | - | - | - | + | + | + | + | + | + | + | + | + | + | + | + | + | + | + |
| Selenium or a b.a. acid or salt or derivative thereof | - | - | - | - | - | - | + | + | + | + | + | + | + | + | + | + | + | + | + | + | + |
| Glucocorticoid, esp. dex | - | - | - | - | - | - | + | + | + | + | + | + | + | + | + | + | + | + | + | + | + |

### Embodiment F1:

| | 0 | 1 | 2 | 3 | 4 | 5 | 6 | 7 | 8 | 9 | 1 0 | 1 1 | 1 2 | 1 3 | 1 4 | 1 5 | 1 6 | 1 7 | 1 8 | 1 9 | 20 etc. |
|---|---|---|---|---|---|---|---|---|---|---|---|---|---|---|---|---|---|---|---|---|---|
| HGF or or b.a. variant or derivative thereof | + | + | + | + | + | + | + | + | + | + | + | + | + | - | - | - | - | - | - | - | - |
| Insulin or or b.a. variant or derivative thereof | - | - | - | - | - | - | + | + | + | + | + | + | + | - | - | - | - | - | - | - | - |
| Transferrin or or b.a. variant or derivative thereof | - | - | - | - | - | - | + | + | + | + | + | + | + | - | - | - | - | - | - | - | - |
| Selenium or a b.a. acid or salt or derivative thereof | - | - | - | - | - | - | + | + | + | + | + | + | + | - | - | - | - | - | - | - | - |
| Glucocorticoid, esp. dex | - | - | - | - | - | - | + | + | + | + | + | + | + | + | + | + | + | + | + | + | + |
| OSM | - | - | - | - | - | - | - | - | - | - | - | - | - | + | + | + | + | + | + | + | + |

### Embodiment F2

| | 0 | 1 | 2 | 3 | 4 | 5 | 6 | 7 | 8 | 9 | 1 0 | 1 1 | 1 2 | 1 3 | 1 4 | 1 5 | 1 6 | 1 7 | 1 8 | 1 9 | 20 etc. |
|---|---|---|---|---|---|---|---|---|---|---|---|---|---|---|---|---|---|---|---|---|---|
| HGF or or b.a. variant or derivative thereof | + | + | + | + | + | + | + | + | + | + | + | + | + | + | + | + | - | - | - | - | - |
| Insulin or or b.a. variant or derivative thereof | - | - | - | + | + | + | + | + | + | + | - | - | - | - | - | - | - | - | - | - | - |
| Transferrin or or b.a. variant or derivative thereof | - | - | - | + | + | + | + | + | + | + | - | - | - | - | - | - | - | - | - | - | - |
| Selenium or a b.a. acid or salt or derivative thereof | - | - | - | + | + | + | + | + | + | + | - | - | - | - | - | - | - | - | - | - | - |
| Glucocorticoid, esp. dex | - | - | - | - | - | - | + | + | + | + | + | + | + | + | + | + | + | + | + | + | + |
| OSM | - | - | - | - | - | - | - | - | - | - | - | - | + | + | + | + | + | + | + | + | + |

### Embodiment H1:

| | 0 | 1 | 2 | 3 | 4 | 5 | 6 | 7 | 8 | 9 | 1 0 | 1 1 | 1 2 | 1 3 | 1 4 | 1 5 | 1 6 | 1 7 | 1 8 | 1 9 | 20 etc. |
|---|---|---|---|---|---|---|---|---|---|---|---|---|---|---|---|---|---|---|---|---|---|
| FGF-4 or b.a. variant or derivative thereof | + | + | + | + | + | + | - | - | - | - | - | - | - | - | - | - | - | - | - | - | - |
| HGF or or b.a. variant or derivative thereof | - | + | + | + | + | + | + | + | + | + | + | + | + | + | + | + | - | - | - | - | - |
| Insulin or or b.a. variant or derivative thereof | - | - | - | + | + | + | + | + | + | - | - | - | - | - | - | - | - | - | - | - | - |
| Transferrin or or b.a. variant or derivative thereof | - | - | - | + | + | + | + | + | + | - | - | - | - | - | - | - | - | - | - | - | - |
| Selenium or a b.a. acid or salt or derivative thereof | - | - | - | + | + | + | + | + | + | - | - | - | - | - | - | - | - | - | - | - | - |
| Glucocorticoid, esp. dex | - | - | - | - | - | - | + | + | + | + | + | + | + | + | + | + | + | + | + | + | + |
| OSM | - | - | - | - | - | - | - | - | - | - | - | - | + | + | + | + | + | + | + | + | + |

As further discussed in the results section, the inventors advantageously realised that differentiation of RLEC may proceed better if the cells are not initially exposed to FGF-4, i.e., if the differentiation protocol does not include exposure to FGF-4. Rather, the cells may be advantageously exposed, as from t=0 to at least HGF or a biologically active variant or derivative thereof.

In a further embodiment of the invention, the cells subjected to sequential exposure to two or more differentiation agents may be further exposed to one or more histone deacetylase (HDAC) inhibitors. The inventors realised that treatment with HDAC inhibitor(s) may further improve the hepatocyte-like properties of the obtained cells (see example 12). However, as also seen in the examples, the differentiation method works advantageously even without the addition of HDAC inhibitors.

Histone deacetylases (hereinafter also referred to as HDAC) represent a class of enzymes that catalyze removal of an acetyl group from the epsilon-amino group of lysine side chains in histones, e.g., histones H2A, H2B, H3 or H4 (Gray et al. 2001. Exp Cell Res 262:75-83). Without being bound by the theory, HDAC are hypothesized to play an important role in regulating gene expression, and thereby also cell proliferation and differentiation, through chromatin remodeling.

Based on sequence similarity, mammalian HDAC can be classified into four distinct families: Class I, including but not limited to HDAC1, HDAC2, HDAC3, HDAC8, and HDAC11; Class II, including but not limited to HDAC4, HDAC5, HDAC6, HDAC7, HDAC9, and HDAC10; and Class III.

The term "histone deacetylase inhibitor" or "HDAC inhibitor" denotes any agent capable of inhibiting the activity of at least one histone deacetylase by, depending on the concentration of such agent, e.g., 10%, 20%, 30%, 40%, 50%, 60%, 70%, 80%, 95% or more. HDAC inhibitors may preferably inhibit both Class I and Class II HDAC. Such agents may take the form of a chemical substance, a pharmaceutical agent or drug, a therapeutically effective oligonucleotide, a specific binding agent, or a fragment or variant of histone deacetylase. Within the present invention, the HDAC inhibitor may preferably be a specific HDAC inhibitor, i.e., an agent that, while inhibiting the activity of at least one HDAC, does not substantially affect other enzyme systems, and wherein the effect(s) on downstream cellular components and functions are a direct consequence of that agent.

Several structural classes of histone deacetylase inhibitors have been identified, comprising naturally occurring as well as synthetic compounds. These include, by means of example and not limitation, benzamides (e.g., MS-27-275, CI-994), sulfonamide-based anilides, straight chain anilides, heterocyclic ketones (e.g., apicidin and its derivatives, FR235222), allyl sulfur compounds (e.g., diallyl sulfide), psammaplins (e.g., psammaplin A), heterocyclic thiols (e.g., depsipeptide, spiruchostatin A), sulfur-containing cyclic peptides, bromoacetamide-based straight chain inhibitors, short-chain fatty acids (e.g., sodium butyrate), pivaloyloxymethyl butyrate, tributyrin, phenylalkanoic acids, valproic acid, phenylbutyrate, N-acetylcystein sulforaphane, cystein sulforaphane, straight chain trifluoromethylketones, alpha-ketoamides, alpha-ketoesters, heterocyclic ketones, cyclic trifluoromethylketones, cyclic pentafluoromethylketones, heterocyclic epoxides (e.g., trapoxin A), depudecin, short-chain fatty hydroxamic acids, trichostatins (e.g., trichostatin A), hybrid polar compound (e.g., suberoyl anilide hydroxamic acid, pyroxamide), benzamide-based hydroxamic acids (e.g., 6-(4-dimethylaminobenzoyl)aminocaproic acid hydroxamide, 5-(4-dimethylaminobenzoyl)-aminovaleric acid hydroxamid), arylketones, amino acid-containing benzamide based hydroxamic acids, indole amide-based hydroxamic acids, aryl-heterocyclic-based hydroxamic acids, aryl-pentadienoic hydroxamic acids, sulfonamide-based hydroxamic acids (e.g., oxamflatin, PXD101), (bi-)aryl-(heterocyclic)-based hydroxamic acids (e.g., SK-7041), NVP-LAQ824, A-161906, aroyl-pyrrole-hydroxyamides, cyclic hydroxamic acid-containing peptides, cyclic hexapeptides, succinimide hydroxamic acids, cystein-based hydroxamic acids, 1,3-dioxanes (e.g., tubacin), phosphorus-based straight chain inhibitors, cyclostellettamines (e.g., cyclic 3-alkyl pyridinium dimers), straight-chain retrohydroxamates, cyclic retrohydroxamates, semicarbazide-based straight chain inhibitors, and thiol-based straight chain inhibitors.

Accordingly, in an embodiment of the present invention, at least one histone deacetylase inhibitor may be chosen from HDAC inhibitors falling into one of the above structural classes. However, it will be appreciated that all presently known HDAC inhibitors, whether falling into one of the above categories or not, may be used in the methods according to the present invention. Also, novel HDAC inhibitors identified in the future can be used in the method according to the present invention. In addition, any HDAC inhibitor may be used as a derivative, e.g., as a biologically acceptable salt, e.g., in order to exert maximum effect in solution, in cells, or in an organism.

In a preferred embodiment, the method may employ Trichostatin A (TSA), which is a potent specific inhibitor of Class I and Class II HDAC *in vitro* and *in vivo* (Yoshida et al. 1990. J Biol Chem 265:17174-17179, 1990). Trichostatin A may be brought in the form of derivates with HDAC inhibitory activity, such as salts, preferably biologically acceptable salts, which may be used in the present method.

In another embodiment, semi-synthetic or synthetic TSA analogues, or derivatives thereof with HDAC inhibitory activity, such as salts, and preferably biologically acceptable salts, may be employed. For example, Jung et al. 1999 (J Med Chem 42: 4669-4679) described the production and properties of several amide based TSA analogues, including, e.g., 6-(4-dimethylaminobenzoyl)-aminocaproic acid hydroxamid. For example, Van Ommeslaeghe et al. 2003 (Bioorg Med Chem Lett 13:1861-1864) described the production of several amide based TSA analogues, among others 5-(4-dimethylaminobenzoyl)-aminovaleric acid hydroxamid. In another example, 5-(4-dimethylaminobenzoyl)-aminovaleric acid hydroxamid is a potent HDAC inhibitor and is metabolically more stable than TSA, when incubated with freshly isolated rat hepatocytes (Elaut G. In vitro hepatoxicity screening of the histone deacetylase inhibitor trichostatin A and its structural analogue 4-ME2N-BAVAH, in Pharmaco-toxicological screening of hydroxamic acid-based histone deacetylase inhibitors in primary hepatocyte cultures, PhD thesis, 2005, chapter 6).

The invention may further employ any or more of HDAC inhibitors listed in the following tables:

| **Jung-1 type compounds with pentanoic spacer** | **Code** |
|---|---|
| 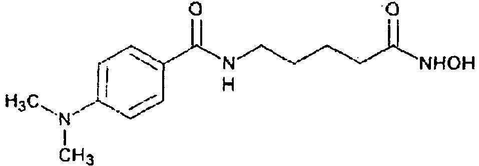 | ***AN-1, AN-2*** |
| 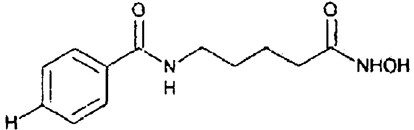 | ***AN-3*** |
| 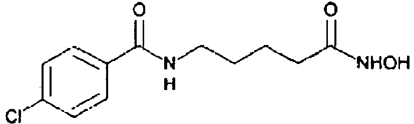 | ***AN-4*** |
| 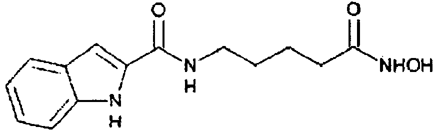 | ***AN-9*** |
| 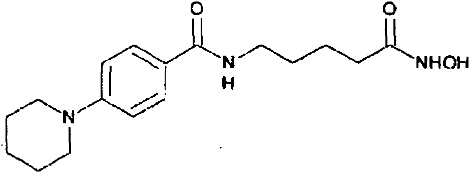 | ***AN-13*** |
| 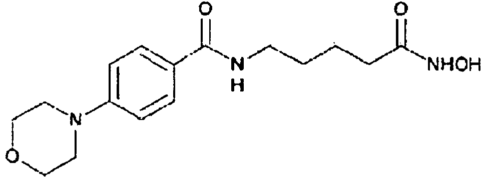 | ***AN-14*** |

| **Jung type compounds with hexanoic spacer** | **Code** |
|---|---|
| 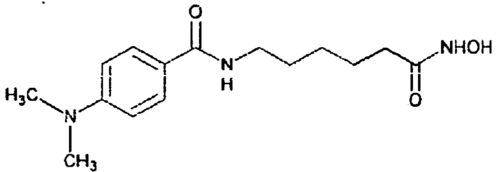 | ***AN-6*** |
| 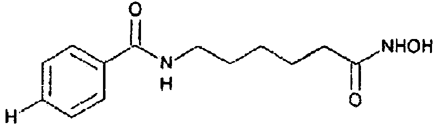 | ***AN-5*** |
| 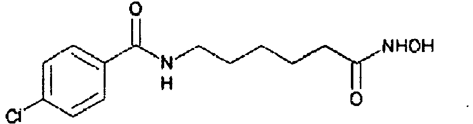 | ***AN-7*** |
| 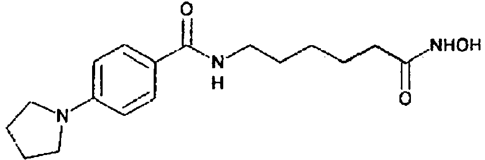 | ***AN-8*** |
| 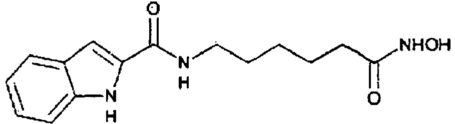 | ***AN-10*** |
| 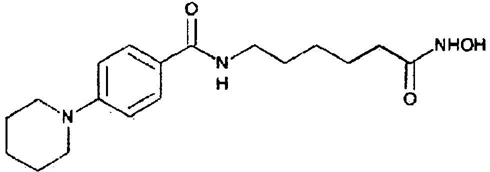 | ***AN-15*** |
| 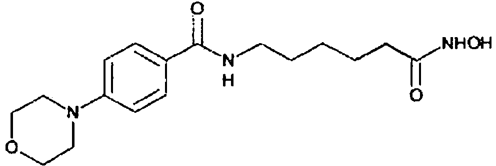 | ***AN-16*** |

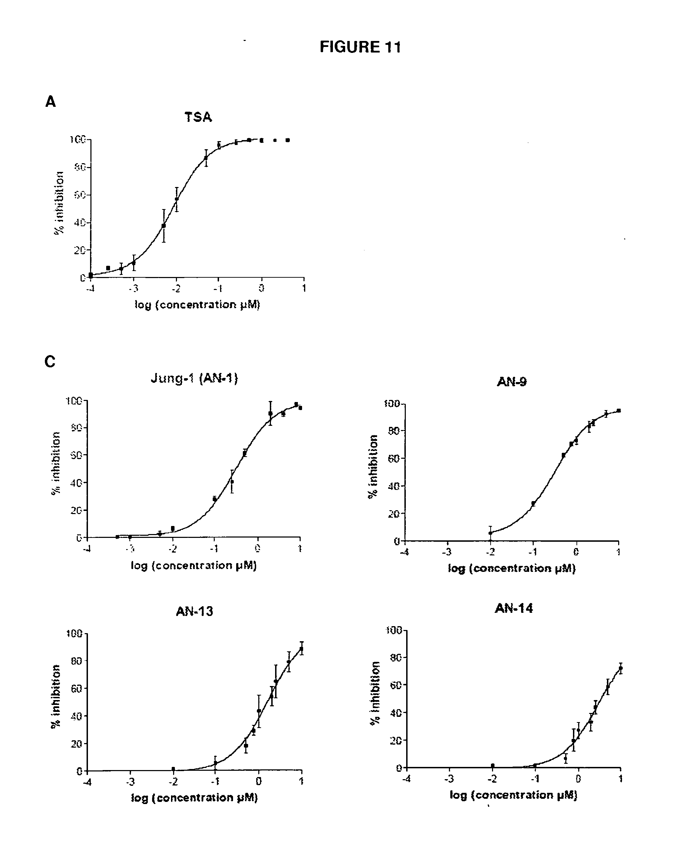

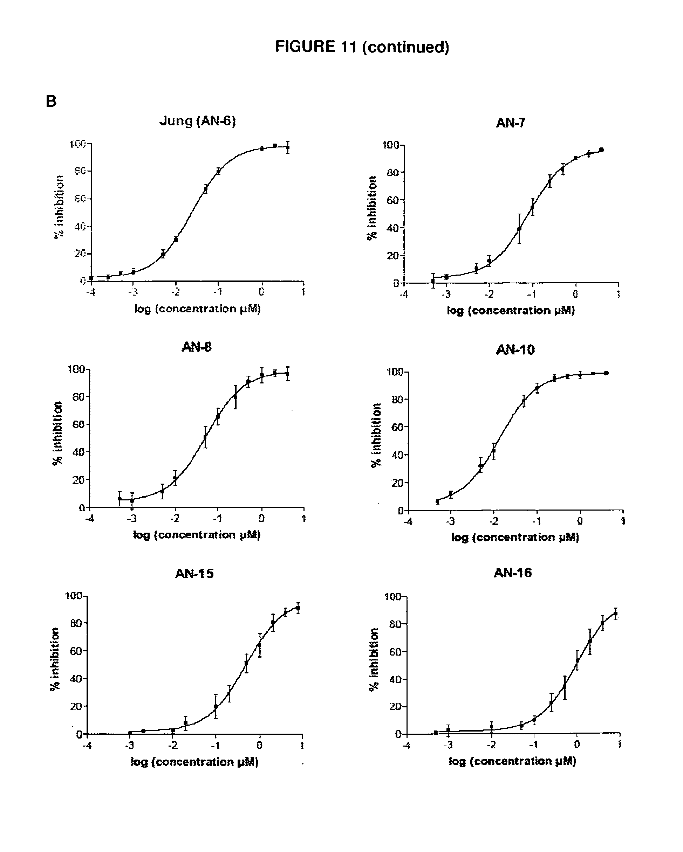

Screening of the HDAC inhibiting potencies of the above compounds was performed in crude rat hepatocyte lysates, prepared from freshly isolated cells, using the Fluor de Lys HDAC inhibition assay as detailed in example 9. The results are shown in Figure 11 and summarized in the following table:

**Table: Estimated IC50 values for tested compounds (n=3 or 4)**

| | **compound** | **IC50 (nM)** |
|---|---|---|
| | TSA | 9 ± 3 |
| ***Jung type*** | Jung* | 24 ± 2 |
| | AN-7 | 84 ± 27 |
| | AN-8 | 55 ± 19 |
| | AN-10 | 14 ± 4 |
| | AN-15 | 500 ± 108 |
| | AN-16 | 1011 ± 310 |
| ***Jung-1 type*** | Jung-1** | 301 ± 28 |
| | AN-9 | 313 ± 50 |
| | AN-13 | 2253 ± 616 |
| | AN-14 | 4487 ± 1822 |

| | | |
|---|---|---|
| *Jung = 6-(4-dimethylaminobenzoyl)-aminocaproic acid hydroxamid (AN-6) **Jung-1 = 5-(4-dimethylaminobenzoyl)-aminovaleric acid hydroxamid (AN-1, 2) | | |

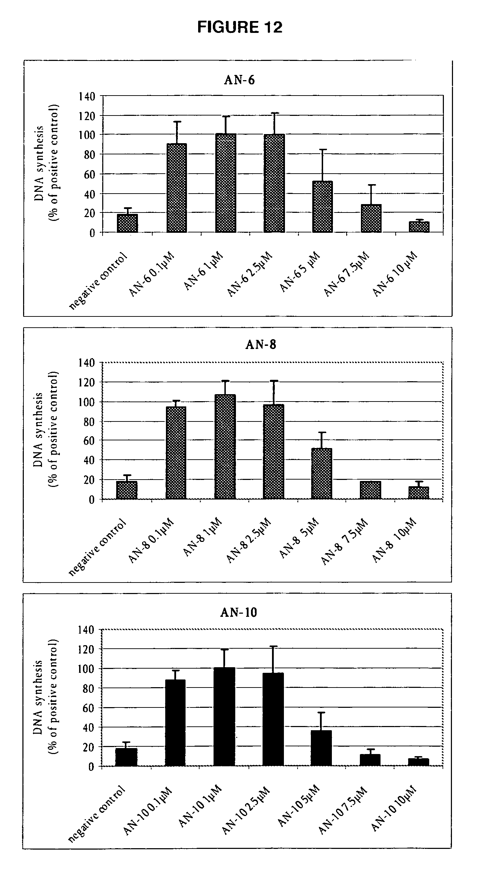

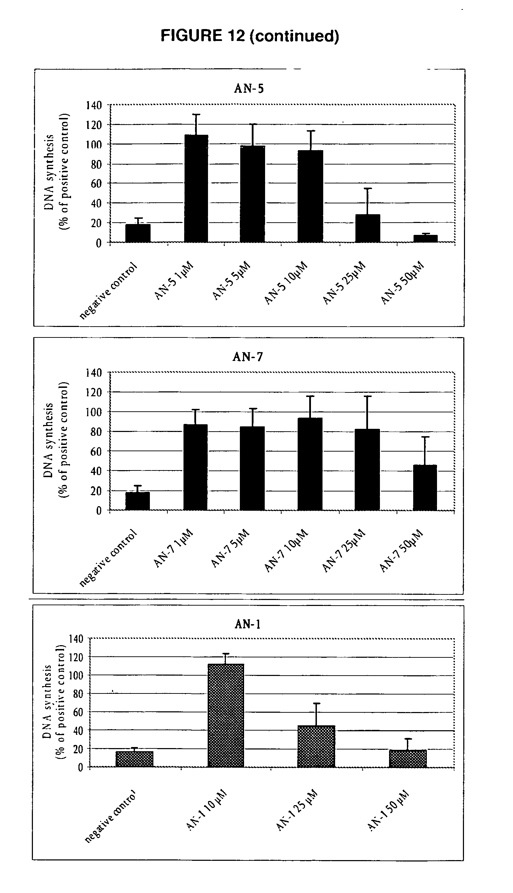

Screening of the anti-proliferative activity of the above compounds (effect on DNA synthesis) was performed as in example 10a. The results are summarised in Figure 12.

The maximal (100%) inhibition of rat primary hepatocyte proliferation has been achieved with following concentrations:

| Compound | Concentration (µM) |
|---|---|
| AN-6 | 10 |
| AN-8 | 7.5 |
| AN-10 | 7.5 |
| AN-5 | 50 |
| AN-1 | 50 |

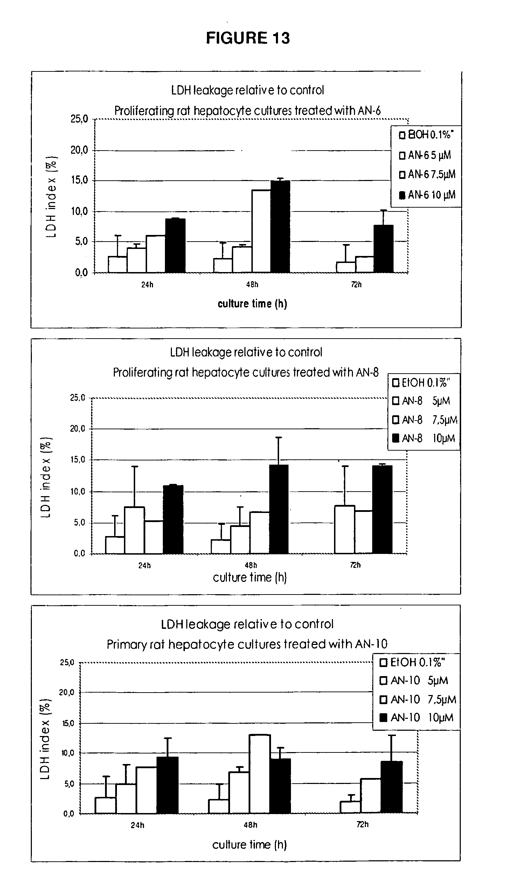

The potential cytotoxicity of the most potent compounds was examined as described in example 10b. Tested concentrations were not cytotoxic (see Figure 13).

The effect of the different 6C-spacer compounds on the expression of key cell cycle proteins was investigated as well in order to identify the location of the cell cycle arrest.

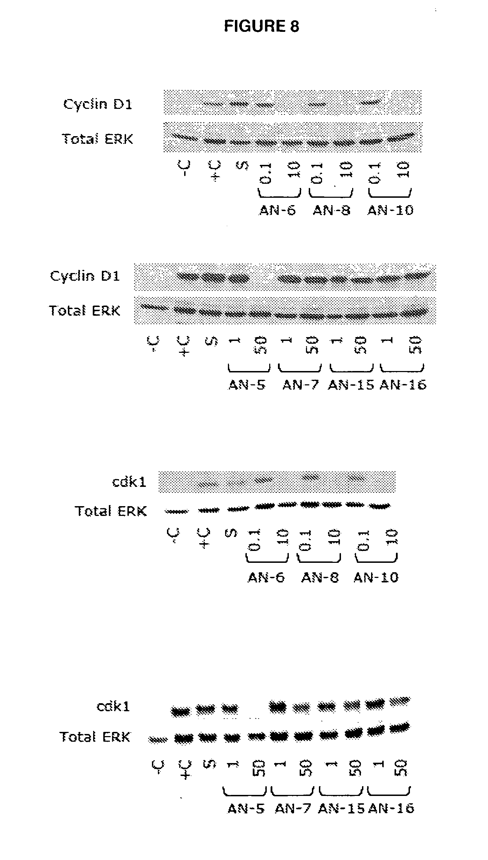

Two cell cycle markers were studied as detailed in example 11 a (see Figure 8 for results): (a) cyclin D1, this protein is typically expressed when hepatocytes were able to pass the restriction point located in the mid-late G1 phase; and (b) cdk1, which is a marker for the S/G2/M phase (DNA synthesis takes place in the S phase).

These experiments reveal that AN-6, AN-8 and AN-10, being identified as particularly potent compounds of the 6C-spacer series, induced an early G1 cell cycle arrest, prior to the restriction point (absence of cyclin D1 and cdk1 protein expression).

From the 5C-spacer series, at least AN-5 was able to induce a similar early G1 cell cycle arrest. The other compounds did not seem to reduce the expression of cyclin D1 (indicating that cells progressed beyond the mid-late G1 restriction point) and only slightly decreased the expression of cdk1, in this experiment.

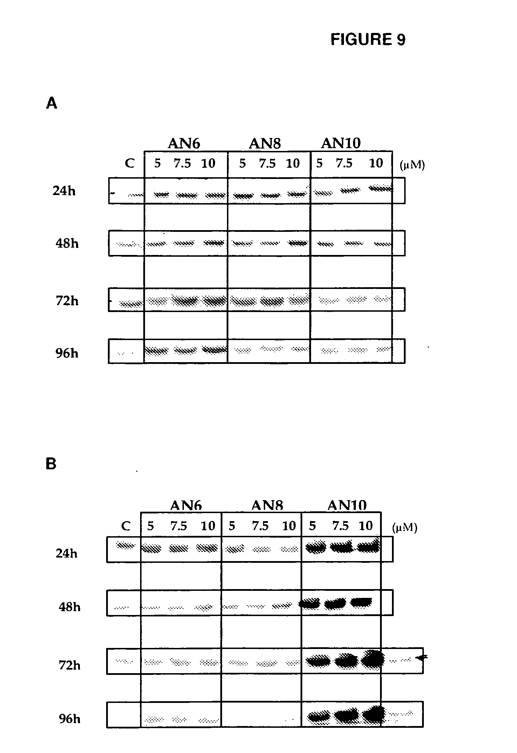

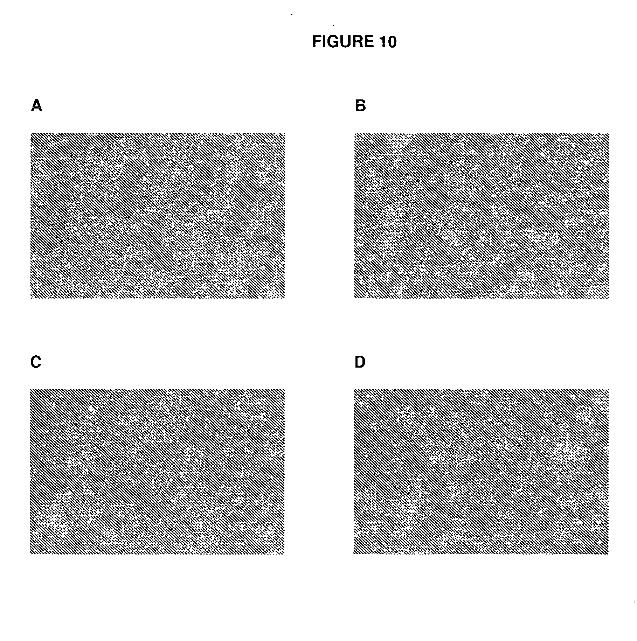

Furthermore, pro-differentiating effect of AN-6, AN-8 and AN-10 was examined as described in example 11 b. Namely, the expression of phase I biotransformation markers, CYP 450 isoforms was examined. Among the analogues tested, AN-6 and AN-10 demonstrated remarkable ability to maintain CYP2 B1 and CYP1 A1 expression, respectively, during the entire culture period. In comparison, the expression of latter proteins was lost in controls (untreated cells). See Figure 9 for the results. Additional analysis of hepatocyte morphology by light microscopy revealed that even the highest (10µM) concentration of tested compounds was well tolerated as demonstrated (see Figure 10).

Accordingly to the above described results, and with reference to examples 9, 10 and 11, the present invention may preferably employ any, more or all of AN- 5, 6, 8, 9 and 10.

Further, the present invention also relates to the above HDAC inhibitors as such, and the uses thereof in any applications.

In the present method, cells may be typically exposed to one or more HDAC inhibitors through the inclusion of the latter in the media, in which the cells are cultured. The one or more HDAC inhibitors can be included at a concentration required to elicit a biological response thereto. A skilled person will understand that an optimal concentration or range thereof may differ for each HDAC inhibitor and may depend on several factors, e.g., the potency of the inhibitor, its metabolic stability in cells and its selectivity for a particular HDAC isoenzyme. A skilled person may be knowledgeable of concentrations needed to elicit biological response in cells for many commonly used HDAC inhibitors. In addition, he may determine the required concentration for these and other HDAC inhibitors by measuring, e.g., the extent of histone acetylation, when applying such HDAC inhibitors to cells. HDAC inhibitors may be typically included in a medium at a final concentration of between 10nM and 500µM and more preferably at between 100nm-50µM.

The cells may be exposed to the one or more HDAC inhibitors during a time interval the onset of which is preferably between *t*=96 hours and *t*=480 hours, more preferably at about *t*=144 hours or later, e.g., at about *t*=192 hours, *t*=240 hours, *t*=288 hours, *t*=360 hours, *t*=432 hours, or *t*=480 hours. In alternative embodiments, the cells may be exposed to the one or more HDAC inhibitor during a time interval the onset of which is between *t*=0 hours and *t*=96 hours, such as, *e.g.*, at about *t*=0 hours, at about *t*=8 hours, at about *t*=24 hours, at about *t*=48 hours, at about *t*=72 hours or at about *t*=96 hours. In a preferred embodiment, exposure of the cells to the said inhibitor(s) may continue for as long as the cells obtained by the differentiation method are maintained in culture.

As can be appreciated by a skilled person, the choice of the medium in which RLEC are differentiated is in principle not essential to the working of the invention. Hence, a skilled person may optimize different types of media for use in the method. Such media will typically comprise a basal medium formulation as known in the art. Many basal media formulations (available, e.g., from the American Type Culture Collection, ATCC; or from Invitrogen, Carlsbad, California) can be used to culture the primary cells herein, including but not limited to Eagle's Minimum Essential Medium (MEM), Dulbecco's Modified Eagle's Medium (DMEM), alpha modified Minimum Essential Medium (alpha-MEM), Basal Medium Essential (BME), Iscove's Modified Dulbecco's Medium (IMDM), BGJb medium, F-12 Nutrient Mixture (Ham), Leibovitz L-15, DMEM/F-12, Essential Modified Eagle's Medium (EMEM), RPMI-1640, Medium 199, Waymouth's MB 752/1 or Williams' Medium E, and modifications and/or combinations thereof. Compositions of the above basal media are generally known in the art and it is within the skill of one in the art to modify or modulate concentrations of media and/or media supplements as necessary for the cells cultured. In an embodiment, a preferred basal medium formulation may be Williams' Medium E, which is a rich formulation reported to sustain *in vitro* culture of RLEC and hepatocyte-like cells. Other embodiments may employ further basal media formulations, such as chosen from the ones above.

Such basal media formulations contain ingredients necessary for mammal cell development, which are known *per se*. By means of illustration and not limitation, these ingredients may include inorganic salts (in particular salts containing Na, K, Mg, Ca, Cl, P and possibly Cu, Fe and Zn), physiological buffers (e.g., HEPES, bicarbonate), nucleotides, nucleosides and/or nucleic acid bases, ribose, deoxyribose, amino acids, vitamins, antioxidants (e.g., glutathione) and sources of carbon (e.g. glucose, pyruvate, e.g., sodium pyruvate, acetate, e.g., sodium acetate), etc. It will also be apparent that many media are available as low-glucose formulations with or without sodium pyruvate.

For use in culture, basal media can be supplied with one or more further components. For example, additional supplements can be used to supply the cells with the necessary trace elements and substances for optimal growth and expansion. Further antioxidant supplements may be added, e.g. ascorbic acid. While many basal media already contain amino acids, some amino acids may be supplemented later, e.g., L-glutamine, which is known to be less stable when in solution. A medium may be further supplied with antibiotic and/or antimycotic compounds, such as, typically, mixtures of penicillin and streptomycin, and/or other compounds, exemplified but not limited to, amphotericin, ampicillin, gentamicin, bleomycin, hygromycin, kanamycin, mitomycin, mycophenolic acid, nalidixic acid, neomycin, nystatin, paromomycin, polymyxin, puromycin, rifampicin, spectinomycin, tetracycline, tylosin, and zeocin.

Lipids and lipid carriers can also be used to supplement cell culture media. Such lipids and carriers can include, but are not limited to cyclodextrin, cholesterol, linoleic acid conjugated to albumin, linoleic acid and oleic acid conjugated to albumin, unconjugated linoleic acid, linoleic-oleic-arachidonic acid conjugated to albumin, oleic acid unconjugated and conjugated to albumin, among others. Albumin can similarly be used in fatty-acid free formulations.

In a preferred embodiment, RLEC when maintained undifferentiated are in a medium supplemented with mammalian serum, preferably foetal bovine serum or foetal calf serum, which contains cellular factors and components that are necessary for viability and expansion. The use of suitable serum replacements is also contemplated. Heat inactivation of such serum as known in the art, e.g., at 56°C for 30 to 60min, e.g., 30min, with steady mixing, is contemplated for culturing RLEC without differentiation, and optionally also filter sterilisation.

In a preferred embodiment, in the course of the present differentiation method, the RLEC are substantially not exposed to serum components, e.g., FBS or FCS. For example, as of *t*=0 hours and onwards, cells may be placed into a medium not comprising serum component. Alternatively, the cells may be placed into a medium not comprising a serum component as of between *t*=0 hours and *t*=48 hours, and preferably as of between *t*=0 hours and *t*=24, e.g., at about *t*=12 hours or *t*=24 hours and onwards. Such short exposure to serum in the initial stages of the method may serve to accommodate the cells to conditions without serum; advantageously, concentration of serum may be decreased, e.g., to less than 5%, e.g. to about 2%, during these initial stages.

In another embodiment, it may be preferred that a culture of cells of an RLEC line is, at the start of the present method (*t*=0 hours), at least 50%, e.g., at least 60%, preferably at least 70%, e.g., at least 80%, more preferably at least 90%, e.g., at least 95% or even substantially 100% confluent. The term "confluence" as used herein refers to a density of cultured cells in which the cells contact one another covering substantially all of the surfaces available for growth (i.e., fully confluent). The inventors found that higher confluence of these cells leads to better differentiation using the present method.

In the present invention, RLEC are plated for culturing and differentiation onto an adherent substrate. In general, adherent substrates may be any substantially hydrophilic substrate. As known in the art, culture vessels, e.g., culture flasks, well plates, dishes, or the like, may be usually made of a large variety of polymeric materials, including, but not limited to polyacrylates, polymethylacrylates, polycarbonates, polystyrenes, polysulphones, polyhydroxyacids, polyanhydrides, polyorthoesters, polyphosphazenes, polyphosphates, polyesters, nylons or mixtures thereof, etc. Generally, culture vessels made of such materials are surface treated after moulding in order to provide for hydrophilic substrate surfaces and thereby enhance the likelihood of effective cell attachment. Surface treatment may take the form of a surface coating, or may involve the use of directed energy at the surface with the intention of generating chemical groups on the polymer surface. These chemical groups will have a general affinity for water or otherwise exhibit sufficient polarity to permit stable adsorption to another polar group. These functional groups lead to hydrophilicity and or an increase in surface oxygen and are properties recognized to enhance cell growth on so modified substrate surfaces. Such chemical groups may include groups such as amines, amides, carbonyls, carboxylates, esters, hydroxyls, sulfhydryls and the like. Examples of directed energy include atmospheric corona discharge, radio frequency (RF) vacuum plasma treatment, and DC glow discharge or plasma treatment (e.g., US 6,617,152). Current standard practices for growing adherent cells may involve the use of defined chemical media with addition of bovine or other animal serum. The added serum, besides providing nutrients and/or growth promoters, may also promote cell adhesion by coating the treated plastic surface with a layer of matrix to which cells can better adhere.

An alternative substrate surface compatible with cell adhesion may be glass, optionally surface treated to introduce functional groups, e.g., as listed above, to increase the hydrophilicity thereof.

Other adherent substrate surfaces may be generated via surface coating, e.g., coating of the polymeric or treated polymeric surfaces as above. In a non-limiting example, the coating may involve suitable poly-cations, such as, e.g., poly-omithine or poly-lysine.

In another example, preferred coating, and accordingly the substrate, comprises one or more components of extracellular matrix, e.g., the ECM proteins fibrin, laminin, collagen, preferably collagen type 1, glycosaminoglycans, e.g., heparin or heparan sulphate, fibronectin, gelatine, vitronectin, elastin, tenascin, aggrecan, agrin, bone sialoprotein, cartilage matrix protein, fibrinogen, fibulin, mucins, entactin, osteopontin, plasminogen, restrictin, serglycin, SPARC/osteonectin, versican, thrombospondin 1, or cell adhesion molecules including cadherins, connexins, selectins, by themselves or in various combinations.

A particularly preferred embodiment includes coating consisting of or comprising collagen, esp. collagen type 1. Such coating may be particularly preferred during the differentiation protocol, since collagen, esp. collagen type 1, has been shown to aid maintenance of hepatocyte function, differentiation state and hepatic gene transcription. Preferably, collagen type I may be used at a concentration of about 100µg/ml.

The specification further describes rat hepatocyte-like cells, or hepatocyte-like cells from other species including man, obtainable or directly obtained using the herein described methods of the invention from cells of an RLEC line, and in particular hepatocyte-like cells having properties as detailed elsewhere in this specification.

The specification describes hepatocyte-like cells from other species obtainable or directly obtained using a sequential differentiation protocol from LEC cells originating from such organisms, and isolated cell populations comprising such cells.

The specification describes an isolated cell population comprising hepatocyte-like cells, obtainable or directly obtained using the methods of the invention. Such cell population may comprise at least 60%, e.g., at least 65%, preferably at least 70%, e.g., at least 75%, more preferably at least 80%, e.g., 85%, even more preferably at least 90%, e.g., 95% or even at least 96%, at least 97%, at least 98% or at least 99% of hepatocyte-like cells, esp. as defined above.

The said hepatocyte-like cells or a cell population comprising such, or the progeny thereof, may be used in pharmacological, toxicological, screening or testing, therapeutic, diagnostic, and further applications, including but not limited to:
- preparation of bio-artificial liver devices and liver assist devices,
- *in vitro* and animal models of toxicology, pharmacology, or pharmacogenetics,
- testing of new drug metabolism or toxicity,
- liver cell transplantation in order to treat liver metabolic deficiencies, liver degenerative diseases or fulminant liver failure, liver infections diseases, etc.

Accordingly, the specification describes rat hepatocyte-like cells, or hepatocyte-like cells from other species including man, obtainable or directly obtained using the herein described methods, or a cell population comprising such as defined above, or the progeny thereof, for use in therapy and/or for the manufacture of a medicament for the treatment of liver diseases. Such diseases may include disorders affecting liver tissue, diseases affecting the hepatocyte viability and/or function are specifically contemplated, and may represent, e.g., inborn errors, the effect of a disease condition, the effect of trauma, toxic effects, viral infections, etc.

The specification also describes a method for preventing and/or treating liver disease, comprising administration of rat hepatocyte-like cells, or hepatocyte-like cells from other species including man, obtainable or directly obtained using the herein described methods, or a cell population comprising such as defined above, or the progeny thereof, to a subject in need of such treatment. The term "subject" covers animals, preferably mammals, and specifically includes human subjects. Administration is preferably in a therapeutically effective amount, i.e., generally an amount which provides a desired local or systemic effect and performance.

Rat hepatocyte-like cells, or hepatocyte-like cells from other species including man, obtainable or directly obtained using the herein described methods, or a cell population comprising such as defined above, or the progeny thereof, may be administered at a site of liver lesion or at a site from which such cells can migrate to the site of the lesion (e.g. administration to spleen, portal vein, liver pulp, etc., e.g., by injection).

The specification describes a pharmaceutical composition comprising the rat hepatocyte-like cells, or hepatocyte-like cells from other species including man, obtainable or directly obtained using the herein described methods, or a cell population comprising such as defined above, or the progeny thereof.

The specification describes a tissue-engineered organ, or portion, or specific section thereof, a tissue engineered device comprising a tissue of interest and optionally, cytokines, growth factors, or differentiation factors, wherein the cells of the invention are used to generate tissues, esp. liver tissue, esp. tissues comprising hepatocytes. Tissue-engineered organs can be used with a biocompatible scaffold to support cell growth in a three-dimensional configuration, which can be biodegradable. Tissue-engineered organs generated from the cells of the present invention can be implanted into a subject in need of a replacement organ, portion, or specific section thereof. The specification describes the use of the hepatocyte-like cells of the invention as part of a bioreactor, e. g. a liver assist device.

Further, the rat hepatocyte-like cells, or hepatocyte-like cells from other species including man, obtainable or directly obtained using the herein described methods, or a cell population comprising such as defined above, or the progeny thereof may be useful as biological components of detoxification devices such as liver perfusion or liver assist devices. A conventional liver assist device includes a rigid, plastic outer shell and hollow semi-permeable membrane fibres which are seeded with rat hepatocyte-like cells, or from other species including man, obtainable or directly obtained using the herein described methods, or a cell population comprising such as defined above, or the progeny thereof. The fibres may be treated with collagen, lectin, laminin, or fibronectin, for the attachment of cells or left untreated. Body fluid is perfused through the device for detoxification according to well known procedures and then returned to the patient. An example of an LAD suitable for the cells of the present invention is described in International Patent Publication Serial Number PCT US00/15524.

As a tool for the drug testing and development process, the rat hepatocyte-like cells, or hepatocyte-like cells from other species including man, obtainable or directly obtained using the herein described methods, or a cell population comprising such as defined above, or the progeny thereof could be used to assess changes in gene expression patterns caused by drugs being considered for development. The changes in gene expression pattern from potential drugs could be compared with those caused by drugs known to affect the liver. This would allow a pharmaceutical company to screen compounds for their effect on the liver earlier in the development process, saving time and money. Currently, pharmaceutical companies have difficulty obtaining a consistent supply of functional liver cells for toxicity testing. The methods and cells of the present invention answer this need.

The specification describes the rat hepatocyte-like cells, or hepatocyte-like cells from other species including man, obtainable or directly obtained using the herein described methods, or a cell population comprising such as defined above, or the progeny thereof for use in assays of toxicity. The use of differentiated cells may be preferred in certain assays of toxicity, as such cells more closely resemble the cell types present in the tissues and organs of an organism. These differentiated cells will be very useful in assays of toxicity performed *in vitro,* i.e., using cultured cells or suspensions of cells. Such *in vitro* assays examine the toxicity to cultured cells or suspended cells of compounds or compositions, e.g. chemical, pharmaceutical, cosmetic, biocidal or biological compounds and food additives or compositions, or biological agents. In this context, a particular compound or composition may be considered toxic or likely toxic, if it shows a detrimental effect on the viability of cells or on one or more aspect of cellular metabolism or function. Typically, the viability of cells *in vitro* may be measured using colorimetric assays, such as, e.g., the MTT (or MTT derivative) assays or LDH leakage assays, or using fluorescence-based assays, such as, e.g., the Live/Dead assay, CyQuant cell proliferation assay, or assays of apoptosis. Other assays may measure particular aspects of cellular metabolism or function. Accordingly, described are the rat hepatocyte-like cells, or from other species including man, obtainable or directly obtained using the herein described methods, or a cell population comprising such as defined above, or the progeny thereof for use in assays of toxicity. Moreover, because such chemical compounds or compositions may also be comprised in a sample obtainable from the environment, described are differentiated cells for use in assays of ecological toxicity.

Further described are the rat hepatocyte-like cells, or hepatocyte-like cells from other species including man, obtainable or directly obtained using the herein described methods, or a cell population comprising such as defined above, or the progeny thereof for use in assays of carcinogenicity. The use of differentiated cells may be preferred in certain assays of carcinogenicity, as such cells more closely resemble the cell types present in the tissues and organs of an organism. These differentiated cells will be very useful in assays of both genotoxic and non-genotoxic (i.e., epigenetic) carcinogenicity. These differentiated cells will be very useful in assays of carcinogenicity performed *in vitro,* i.e., using cultured cells or suspensions of cells. Such *in vitro* assays examine the carcinogenicity to cultured cells or suspensions of cells of compounds or compositions, e.g. chemical, pharmaceutical, cosmetic, biocidal or biological compounds and food additives or compositions, or biological agents. In this context, a particular compound or composition may be considered carcinogenic or likely carcinogenic, if it induces neoplastic transformation of the cells, or induces phenotypic changes in the cells that may be predictive of such neoplastic transformation, or induces genetic or metabolic changes that may potentially cause such neoplastic transformation. Such phenotypic changes in the cells may, by means of example but not limitation, comprise morphological transformation, increased proliferation, dedifferentiation, independence of attachment, removal of contact inhibition of cells grown in monolayers, or expression of specific marker proteins. Such genetic changes in the cells may, by means of example but not limitation, comprise DNA damage, chromosomal aberrations, such as chromosomal rearrangements, alterations in chromosome number (aneuploidy), or karyotype aberrations, gene mutations, such as point mutations, deletions or insertions. Compounds or compositions that cause this kind of genetic changes are often referred to as mutagenic or mutagens. Accordingly, the cells provided by the present invention will be very useful in assays of mutagens, i.e., in assays of mutagenicity. For the purposes of mutagenicity testing, the cells of the present invention may but need not be genetically altered. For example, the cells may comprise a transgene, the product of which increases their sensitivity to a particular proliferation-inhibiting agent. Consequently, genetic alterations in some cells removing the expression of such transgene would release these cells from this inhibition. Mutagenicity may then be assessed by methods of scoring such cells. Moreover, because such chemical compounds or compositions may also be comprised in a sample obtainable from the environment, described are also provides differentiated cells for use in assays of ecological carcinogenicity and ecological mutagenicity.

Further disclosed are the rat hepatocyte-like cells, or hepatocyte-like cells from other species including man, obtainable or directly obtained using the herein described methods, or a cell population comprising such as defined above, or the progeny thereof for use in assays of biotransformation. The pharmacokinetics of a candidate therapeutic belongs to factors determining its usefulness. For example, low metabolic stability may result in poor availability and high clearance of such candidate therapeutic. Moreover, while in most cases biotransformation of drugs results in bio-inactivation, it is also possible that pharmacologically active, reactive, or toxic intermediates and metabolites are produced. Therefore, the biotransformation of a new chemical entity, such as a drug or a candidate therapeutic, needs to be investigated as early in the drug discovery process as possible. The liver represents the major site of xenobiotic biotransformation and/or detoxification of a wide variety of foreign compounds, including many therapeutic agents, and the pharmacokinetics of a candidate therapeutic are heavily influenced by their biotransformation in the liver. Hepatocyte cultures represent one *in vitro* system capable of integrated biotransformation. Freshly isolated hepatocytes exhibit biotransformation capacities close to those observed *in vivo,* but can only be used in *in vitro* assays of biotransformation for about 5-6 hours. Monolayer cultures of primary hepatocytes may be used in such assays for about 2-4 days. Cells showing phenotypical features of hepatocytes produced by the *in vitro* differentiation method of the present invention, may provide an alternative assay system to study the biotransformation of various compounds or compositions, e.g. chemical, pharmaceutical, cosmetic, biocidal or biological compounds and food additives or compositions, as the outcomes of such assays may prove highly predictive of the biotransformation of the respective various compounds or compositions by primary hepatocytes *in vivo* and/or *in vitro.* Within relation to biotransformation, such differentiated cell will ideally have a biotransformation capacity, i.e., either phase I or phase II, or both phase I and phase II biotransformation capacities, sufficiently comparable to the hepatocytes *in vivo* or to freshly isolated primary hepatocytes. The cells of the present invention will be useful in assays of biotransformation performed *in vitro,* i.e., using adherent cells or suspensions of cells.

The specification also describes a biochemical extract of the rat hepatocyte-like cells, or from other species including man, obtainable or directly obtained using the herein described methods, or a cell population comprising such as defined above, or the progeny thereof. Such extract may primarily contain, by means of example but not limitation, the cytosolic fraction, the nuclear fraction, the cytoplasmic membrane fraction, the nuclear membrane fraction, the microsomal fraction, the mitochondrial fraction, the endoplasmatic reticulum fraction, the Golgi apparatus fraction, the lysosomal fraction, the cytoskeletal fraction, etc. Such extract may comprise cellular proteins, wherein the function of said proteins may or may not be preserved in said extract. Depending on the desired application for the biochemical extract, the choice of the appropriate extracted fraction, as well as the methods suitable to obtain that fraction will be known to the one skilled in the art. One preferred application for the extract may be its use in biotransformation assays.

Further described is a microsomal extract of the rat hepatocyte-like cells, or of hepatocyte-like cells from other species including man, obtainable or directly obtained using the herein described methods, or a cell population comprising such as defined above, or the progeny thereof. Microsomal extracts or suspensions will contain the smooth endoplasmatic reticulum of the differentiated cells and may be used in biotransformation assays to evaluate, for example, phase I oxidation reactions, cytochrome P450 (CYP)-dependent inhibitory drug-drug interactions and the importance of genetic polymorphisms in biotransformation. Methods to obtain microsomal extracts or suspensions for use in biotransformation assays, and methods of carrying out biotransformation assays using such extracts are well known to the person skilled in the art.

The invention will now be illustrated by means of the following examples, which do not limit the scope of the invention in any way.

### EXAMPLES

### Example 1: A detailed method for isolating RLEC lines according to the invention

### Materials:

1. Equipment includes two sterile 100-mL bottles, two sterile nylon filters (80 µm) in funnels, a sterile glass Petri dish of 10 cm diam, a sterile magnet, sterile plastic 50 mL-centrifuge tubes, sterile plastic Petri dishes of 4 cm diam, a fine platinum-needle, sterile volumetric pipets, sterile Pasteur pipets, laminar-airflow cabinet, an incubator (37°C, water jacketed, humidified atmosphere of 95% air and 5 % CO₂), a thermostated bath (37°C), a centrifuge and a phase-contrast inverse light microscope.
2. Sterile phosphate-buffered saline (PBS), pH 7.65, (1 L): 0.28 % (w/v) NaCl; 0.31 % (w/v) Na₂HPO₄.12H₂O; 0.02% (w/v) KCI; 0.02 % (w/v) KH₂PO₄ in Millipore quality water, sterilized by passing through a 0.22-µm filter.
3. Trypsin solution: 0.25 % trypsin (from porcine pancreas, 12,700 U/mg protein) in PBS, sterilized by passing through a 0.22-µm filter (approx 67 mL / g liver).
4. Williams' E medium, can be stored for 6 months at 4°C.
5. 200 mL Williams' E medium for RLEC supplemented with 7.3 IU/mL benzyl penicillin; 50 µg/mL streptomycin sulfate; 50 µg/mL kanamycin monosulfate and 10 µg/mL sodium ampicillin and 0.1 mg/mL L-glutamine (WM). This medium is sterilely prepared and can be stored for 7 days at 4°C.
6. 500 mL WM supplemented with 10 % (v/v) FBS (WMF). This medium is sterilely prepared and can be stored for 7 days at 4°C.

### Procedure:

1. Weigh a sterile, 100-mL bottle with lid containing 50 mL sterile PBS.
2. Decapitate 10 Sprague-Dawley rats of 8-10 d old;
3. Open the abdominal walls of the animals and remove the liver.
4. Collect the livers in the 50 mL PBS.
5. Reweigh the bottle; the difference between this weight and that determined in item 1 is the mass of collected liver.
6. Remove the PBS and transfer the livers to a sterile glass Petri dish of 10 cm diam.
7. Add 8 mL sterile PBS and cut the livers into small pieces.
8. Transfer these to the 0.25 % (w/v) trypsin solution (20 mL/g collected liver) in a 100-mL sterile bottle, add a sterile magnet, seal the bottle and let stir for 10 min on a magnetic stirrer.
9. Leave for a few min. -
10. Remove the trypsin solution carefully with a 10-mL sterile pipette.
11. Add 20 mL trypsin solution/g collected liver and stir for 15 min.
12. Leave for a few min and filter the supernatant through an 80-µm nylon filter into a 50-mL plastic centrifuge tube (save the material that does not pass through the filter).
13. Centrifuge the filtrate for 5 min at 626g.
14. Remove the supernatant, leaving approx 10 mL above the pellet.
15. Dilute the pellet to 35 mL with WM and resuspend.
16. Centrifuge for 5 min at 626 g.
17. Remove the supernatant completely and resuspend the pellet in 1.66 mL WM per g collected liver (=A).
18. To the liver material that did not pass through the filter (step 12), add 60 mL trypsin solution and stir for 15 min.
19. Repeat steps 12 to 17 (=B).
20. Combine A and B together. If an aggregate forms, re-filter.
21. Prepare cultures of four different dilutions. For example, for 3 g collected liver in a total volume of 10 mL WM (= A+B), place 0.25, 0.50, 0.75 and 1.00 mL A+B mixture into four different 4 cm diam Petri dishes. Dilute each to a total volume of 4.00 mL with WMF.
22. Incubate the cultures for 20 min (= first series).
23. Transfer the medium of the first series of cultures into new Petri dishes, make up to 4 mL with WMF, and incubate for 20 min (= second series).
24. Renew the medium of the first series with 4 mL WMF and incubate these for 24 h.
25. After 20 min, transfer the medium of the second series into new Petri dishes, dilute to 4 mL with WMF and incubate for 20 min (= third series).
26. Renew the medium of the second series with 4 mL WMF and incubate these for 24 h.
27. After 2 h, transfer the medium of the third series into new Petri dishes, dilute to 4mL with WMF and incubate for 24 h (= fourth series).
28. Renew the medium of the third series with 4 mL WMF and place these in the incubator for 24 h.
29. After 24 h, renew the medium of all Petri dishes with WMF.
30. Thereafter, renew the medium every 48 h until colonies of RLEC are visible under the microscope (after a few wk).
31. Scan the Petri dishes every day for fibroblasts. When fibroblasts are present, scrape these off using a heated Platinum-needle.
32. When the colonies are large enough (i.e. >100 cells/colony), transfer the RLEC into new 4 cm diam Petri dishes and let these form new colonies by repeating steps 29 and 30.
33. After 2-3 months, a pure population of good dividing RLEC is obtained. They are further subcultured in 75 cm² culture flasks.

### Example 2: A method for cultivation of RLEC lines without differentiation

### Materia/s:

1. Equipment includes 75 cm² sterile flasks treated for tissue culture, sterile volumetric pipettes, sterile Pasteur pipettes, an incubator (37°C, water jacketed, humidified atmosphere of 95% air and 5% CO₂), a thermostated bath (37°C), laminar-airflow cabinet, and a phase-contrast inverse light microscope.
2. Sterile phosphate-buffered saline (PBS), pH 7.65, (1 L): 0.28 % (w/v) NaCl; 0.31 % (w/v) Na₂HPO₄.12H₂O; 0.02% (w/v) KCI; 0.02 % (w/v) KH₂PO₄ in Millipore quality water, sterilized by passing through a 0.22-µm filter.
3. Sterile trypsin-ethylenediamine tetra-acetic acid (EDTA) solution: 0.05% (w/v) trypsin; 0.53 mM EDTA•4Na in Hanks' Balanced Salt Solution (HBSS) without CaCl₂.6H₂O MgCl₂.6H₂O and MgSO₄.7H₂O.
4. WMF.

### Procedure:

1. Add to a 75 cm² culture flask 10 mL WMF at 37°C.
2. Transfer the RLEC from one Petri dish obtained in example 1 into a culture flask (= replication number 1).
3. Incubate the cells for 24 h.
4. Renew with 10 mL WMF at 37°C.
5. Renew medium thereafter every 2 d with 10 mL WMF until a confluent layer of RLEC is obtained. (approx. 8 d).
6. Remove the medium.
7. Add 5 mL sterile PBS (37°C) per flask, shake gently, and remove the PBS.
8. Add 5 mL sterile trypsin-EDTA (37°C) per flask, shake gently and leave the flask sealed for 5 min at room temperature.
9. Remove the trypsin-EDTA, reseal the flask and place it for approx 15 min in the incubator.
10. Detach the RLEC by tapping the flask. Confirm that the cells have detached by examination under the microscope (i.e., are in floating suspension).
11. Add 20 mL WMF at 37°C per flask and transfer 10 mL cell suspension into two new sterile culture flasks (= replication number 2). Mix gently to spread the cell suspension over the flask.
12. Repeat steps 3-11 until enough confluent layers of RLEC are obtained.

### Example 3: A further method for routine sub-cultivation of RLEC

### Growth and basal cultivation media for RLEC:

### Growth medium:

| | Stock concentration | For 250ml | For 500 ml |
|---|---|---|---|
| Williams' E medium | / | 214.15ml | 428.3ml |
| FBS | 100% | 25ml | 50ml |
| L-glutamine | 200mM | 850µl | 1.7ml |
| Streptomycin sulfate | 5mg/ml | 2.5ml | 5ml |
| Benzyl Penicillin | 733 IU/ml | 2.5ml | 5ml |
| Kanamycin monosulfate | 5mg/ml | 2.5ml | 5ml |
| Sodium ampicillin | 1mg/ml | 2.5m | 5ml |

### Filter with 0.22µm filter and store at 4°C.

### Basal medium:

| | Stock concentration | Final concentration | For 500 ml |
|---|---|---|---|
| Williams' E medium | / | / | 500ml |
| LA-BSA | 100X (100mg/ml) | 1 mg/ml | 5ml |
| Benzyl Penicillin | 20000 IE/ml PBS | 100 IE/ml | 2.5ml |
| Streptomycin sulfate | 20mg/ml PBS | 100µg/ml | 2.5ml |
| L-Ascorbic Acid | 100X (10mM in PBS) | 1X (0.1mM) | 5ml |
| Nicotinamide | 1mg/ml | 4mg/1000ml | 2ml |
| Sodium-pyruvate | 273mg/ml | 27.3mg/l | 500µl |
| L-glutamine | 200mM | 1.623mM | 4.06 |

Filter with 0.22µm filter and store at 4°C, after cytokines are added do not keep medium for more than two weeks.

### Material and products:

Sterile volumetric pipettes; sterile Pasteur pipettes; sterile plastic 50ml centrifuge tubes (Falcon); sterile flasks [75 cm² (T75), 150cm² (T150)] treated for tissue culture (Corning); incubator (37°C, water jacketed, humidified atmosphere of 95% air and 5% CO2); thermostated bath (37°C); laminar-airflow cabinet; phase-contrast inverse light microscope (Nikon); growth medium: 37°C; sterile trypsin/EDTA (0.25%/0.1%; JRH Biosciences): thaw aliquots at 37°C, store at 4°C; sterile PBS: 37°C.

### General remarks:

Divide cells 1:2 after 100% confluence. Check cells with microscope every 24 to 48 hrs.

### Sub-cultivation of RLEC

- remove growth medium, by tilting flask and pipetting from corner.
- add 10ml of sterile 1X PBS (37°C) for 1x T150 and 5ml for 1x T75 to rinse away medium
- shake flasks gently to wet entire growth surface
- remove PBS
- add 5ml of trypsin/EDTA (0.25%/0.1%) (37°C) to a T75 or 10ml to T150, shake gently, and leave the flask sealed for 3-5 min at room temperature.
- remove the trypsin/EDTA, reseal the flask, and place the flask(s) for 15min in the incubator.
- detach the RLEC by tapping the flask; check under microscope to ensure that all cells have detached (i.e. they are in floating suspension)
- add 20ml of growth medium (37°C) per T75 flask and transfer 10ml of cell suspension into 2 new T75 flasks (divide 1:2) or 20ml into 1 new T150 flask.
- mix gently to spread the cell suspension over the T75/T150 flask(s).
- Spray flasks with alcohol and place in incubator (37°C).
- after 24h incubation, remove medium
- add 10ml of new growth medium (37°C) per T75 or 20ml per T150 flask
- thereafter, renew medium every 2 days until RLEC reach 100% confluence

### Example 4: Rat tail collagen coating

### Material and products:

- sterile volumetric pipettes.
- micropipettes 0-1000µl.
- yellow and bleu sterile tips.
- sterile Pasteur pipettes.
- sterile plastic 50ml centrifuge tubes (Falcon).
- sterile 24-well plates treated for tissue culture (Falcon).
- 0.22µm filter
- 20ml syringe
- ice
- laminar-airflow cabinet.
- sterile PBS: cold.
- 100% acetic acid: cold.
- sterile MilliQ water: cold
- 4.5mg/ml sterile rat tail collagen, type I (BD Biosciences 354236): cold.

### Collagen thin coating

Preparation of 0.02N acetic acid: add 115µl of 100% acetic acid per 100ml of sterile milliQ water; filter 0.22µm; store at 4°C

Preparation of 100µg/ml collagen: dilute the stock solution of 4.5mg/ml sterile collagen 45 times in 0.02N acetic acid: For 90ml: 2ml of 4.5mg/ml collagen+98ml of 0.02N acetic acid. Prepare on ice.

Preparation of 50µg/ml collagen: dilute the stock solution of 4.5mg/ml sterile collagen 90times in 0.02N acetic acid: For 90ml: 1ml of 4.5mg/ml collagen+99ml of 0.02N acetic acid. Prepare on ice!

### Collagen thin coating:

- transfer 200-250µl of diluted collagen to a 2cm²-well
- swirl well to cover the whole area
- incubate for 1h at room temperature in laminar air-flow cabinet
- carefully aspirate the remaining solution
- rinse 3 x with PBS to remove acid
- plates may be used immediately or may be air dried; they may be stored at 2-8°C for up to 1 week under sterile conditions.

### Example 5: Hepatic differentiation of RLEC

### Material and products:

- sterile volumetric pipettes.
- sterile Pasteur pipettes.
- sterile 24-well plates treated for cell culture with lid (Falcon)
- incubator (37°C, water jacketed, humidified atmosphere of 95% air and 5% CO₂).
- thermostated bath (37°C).
- laminar-airflow cabinet.
- phase-contrast inverse light microscope (Nikon).
- growth medium: 37°C.
- sterile trypsin/EDTA (0.25%/0.1%; JRH Biosciences): thaw at 37°C, store at 4°C.
- sterile PBS: 37°C.
- growth medium
- basal medium
- collagen type I (BD; source: rat tails): dilute to 1mg/ml in acetic acid

### Hepatocyte differentiation

RLEC at 100% confluence are used for differentiation into hepatocyte-like cells:
- after sub-cultivation, detach cells as described above and suspend all cells derived from 1 T75 and 1 T150 in 3ml and 6ml of new growth medium (37°C), respectively.
- homogenize the cell suspension by pipetting up and down.
- add 420µl growth medium to a 2cm²-well
- transfer 80µl of cell suspension to the middle of a 2cm²-well, precoated with 50-100µg/ml collagen type I
- tap to homogenize cell suspension; check cells under microscope
- incubate ceiis at 37°C in growth medium until ceiis have reached 100% confluence.
- At 100% confluence, remove growth medium and expose cells to basal medium supplemented with liver specific factors, as follows:
   1. days 0-1: 10ng/ml FGF-4
   2. days 1-3: 10ng/ml FGF-4, 20ng/ml HGF
   3. days 3-6: 5ng/ml FGF-4, 30ng/ml HGF, 0.5 x ITS
   4. days 6-9: 30ng/ml HGF, 0.25 x ITS; 0.05µM dexamethasone
   5. days 9-12: 20ng/ml HGF, 0.05µM dexamethasone
   6. days 12-15: 10ng/ml HGF; 10ng/ml OSM, 0.05µM dexamethasone
   7. From day 15 on: 10ng/ml OSM, 0.05µM dexamethasone

This allows for a long-term cultivation of average about 30 days

After 6 days differentiation, 1µM TSA (Sigma) can be added to the differentiation media.

Differentiation media are changed every 3 days

### Growth factors and cytokines

FGF-4: work solution of 10ng/ml; 200µl/50ml medium
HGF: work solution of 20ng/ml; 500µl/50ml medium
ITS: work solution of 1 x; 500µl/50ml medium
Dexamethasone: work solution of 0.05µM; 10µl/50ml medium
OSM: work solution of 10ng/ml; 500µl/50ml medium

### Example 6: Alternative protocols of sequential RLEC differentiation

### Sequential 1

1. days 0-6: 20ng/ml HGF
2. From day 6 on: 20 ng/ml HGF + 1x ITS; 0.05µM dexamethasone

### Sequential 2

1. days 0-6: 20ng/ml HGF
2. days 6-12: 20 ng/ml HGF + 1x ITS; 0.05µM dexamethasone
3. From day 15 on: 10ng/ml OSM, 0.05µM dexamethasone

### Sequential 3

1. days 0-3: 20ng/ml HGF
2. days 3-6: 30ng/ml HGF, 0.5 x ITS
3. days 6-9: 30ng/ml HGF, 0.25 x ITS; 0.05µM dexamethasone
4. days 9-12: 20ng/ml HGF, 0.05µM dexamethasone
5. days 12-15: 10ng/ml HGF; 10ng/ml OSM, 0.05µM dexamethasone
6. From day 15 on: 10ng/ml OSM, 0.05µM dexamethasone

### Example 7: Characterisation of differentiation of RLEC prepared and cultured as in examples 1 - 3 and differentiated as in example 5

### Morphology of undifferentiated RLEC as determined by phase contrast microscope

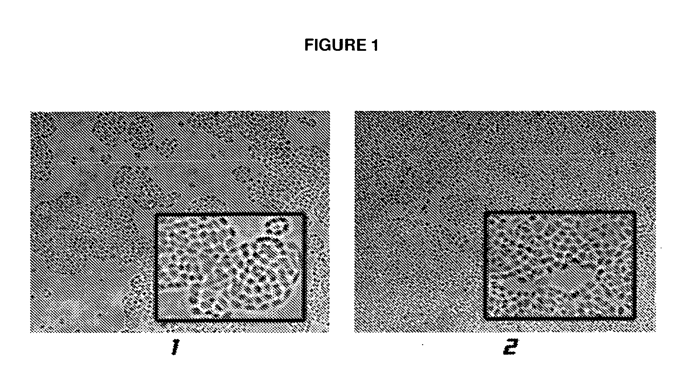

Undifferentiated RLEC display epithelial morphology characterised by an angular, polygonal form and delineated plasma membrane. A representative example of undifferentiated RLEC is shown in Figure 1.

### Characterisation of undifferentiated RLEC using immunofluorescence staining

Immunofluorescence staining of cells was performed according to the protocol detailed in Example 7.

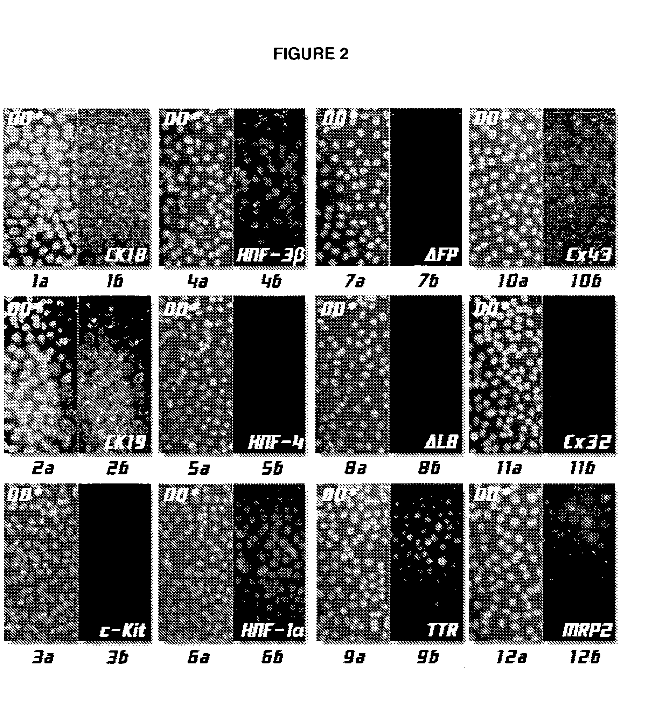

Undifferentiated RLEC show expression of the hepatic marker CK18 and the biliary marker CK19 (Figure 2.1, 2.2), further strongly express Cx43 (Figure 2.10), and also express the liver enriched transcription factors HNF-3beta (Figure 2.4) and HNF-1alpha (Figure 2.6). However, since HNF-3beta is substantially only present in cytoplasm, in particular perinuclear, it is likely not active. Weak expression is observed for the drug transporter MRP2 (Figure 2.12) and the thyroid hormone binding TTR (Figure 2.9), the latter however showing a 'nuclear' localisation unusual for a cytoplasmic protein. The cells in this experiment did not express c-Kit (Figure 2.3).

### Morphological characterisation of hepatocytic differentiation of RLEC

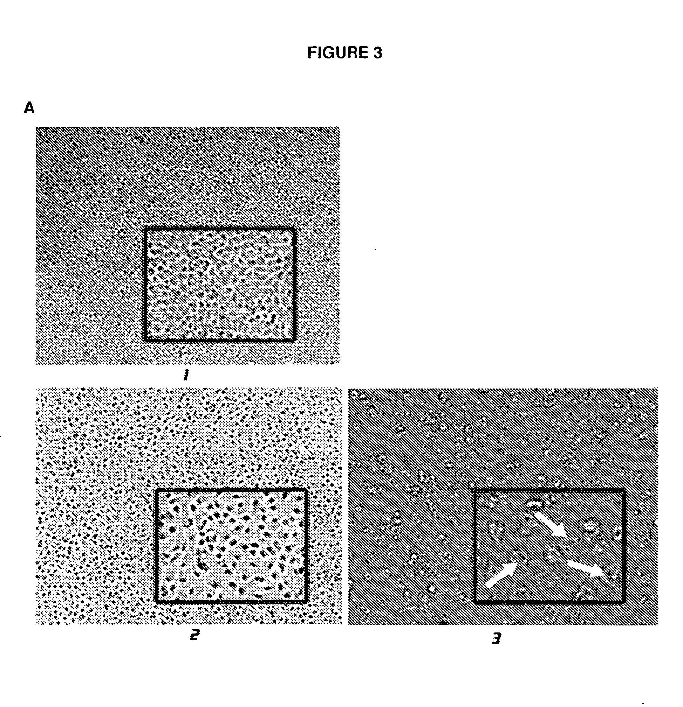

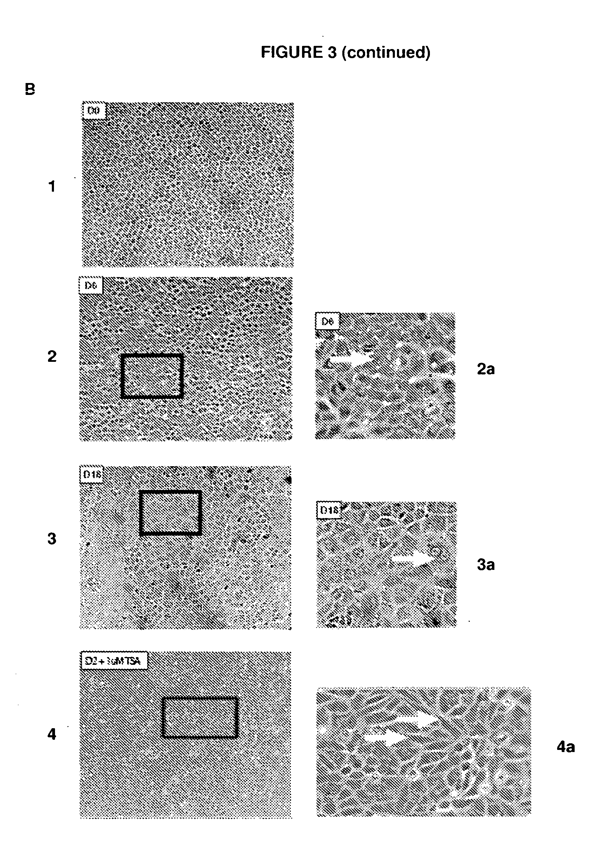

Microscopy demonstrates from day 6 of the differentiation process the appearance of small, epithelium-forming, differentiating cells in the treated RLEC cultures. With advancement of the differentiation, these cells enlarge and acquire a polygonal to cubical form and a clearly visible round nucleus. From day 18, bi-nucleated cells become visible (Figure 3).

Results of a further experiment using the conditions of example 5 are shown in Figure 3B.Fig. 3B.1 shows undifferentiated RLEC at D0. Fig 3B.2 and Fig 3B.2a (magnified frame from Fig 3B.2) show differentiating RLEC on D6 which are already getting more polygonal shape, but are still mononuclear (arrows). Fig 3B.3 and Fig 3B.3a (magnified frame from Fig 3B.3) shows differentiating RLEC on day 18 which have enlarged in size and have a more polygonal to cuboidal shape characterized by 2 nuclei which indicates hepatic maturation (arrows). Experiments with cultivation protocol of example 5 and TSA addition have been also conducted. Here, without limitation to any hypothesis, TSA may seem to enhance an initial "dedifferentiation" period (see below) as the cells get a more fibroblast like shape (Fig. 3B.4 and Fig. 3B.4a, arrows).

### Immunocytochemical characterisation of hepatocytic differentiation of RLEC cells

Immunofluorescence staining of cells was performed according to the protocol detailed in Example 7. Expression of early (HNF-3beta, Cx43, AFP), mid-late (TTR, HNF-4) and late (ALB, CK18, HNF-1&, MRP2 and Cx32) liver specific proteins was followed.

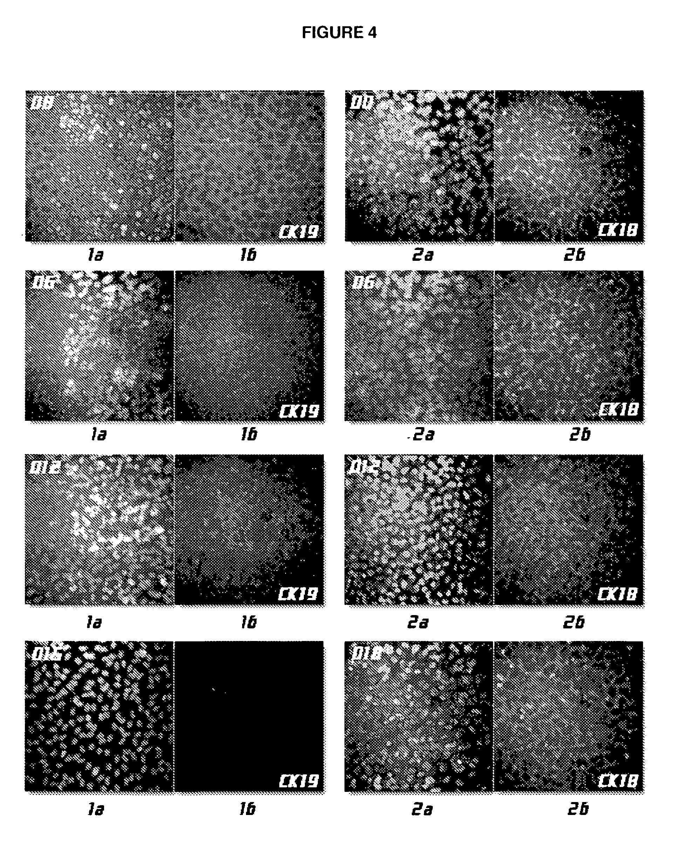

During differentiation the expression of the biliary marker CK19 gradually decreases and is absent from t=15 days (i.e., 15 days after initiation of the differentiation) onwards (Figure 4). On the other hand, the expression of CK18 remains stable during the entire differentiation process (student t-test, p>0.05).

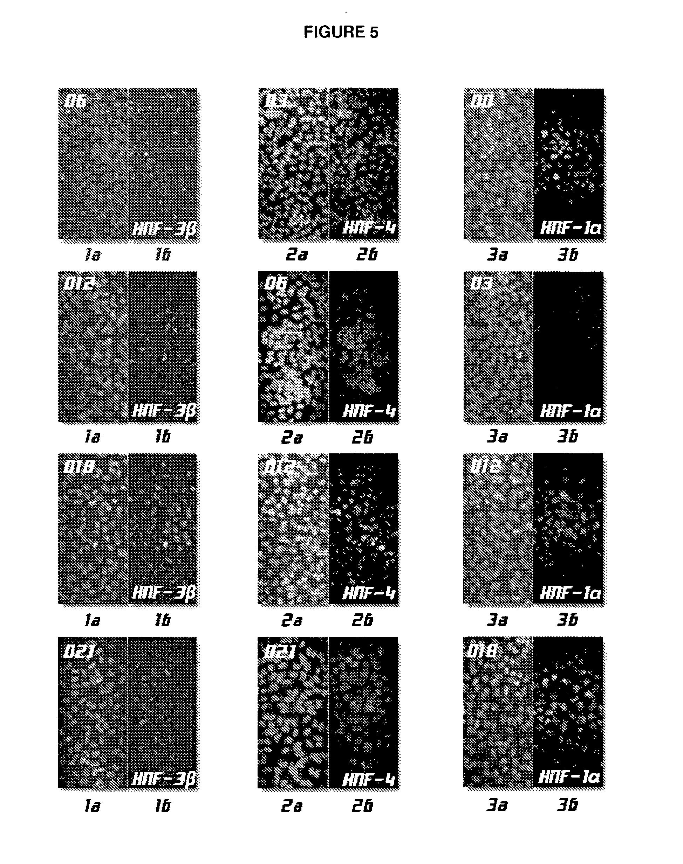

The expression of HNF-3beta also remains constant during the first 12 days of the differentiation process (student t-test, p>0.05) (Figure 5.1). During this period, HNF-3beta translocates from the cytoplasm to the nucleus, which indicates activation of its transcription factor function. The HNF-3beta expression then gradually increases during the subsequent 6 days and again diminishes thereafter. HNF-4 is not expressed by undifferentiated RLEC and its expression is only observed from day 3 of the differentiation process. On day 3, its expression is primarily nuclear, later it becomes mixed nuclear and perinuclear, and on day 21 its is mainly perinuclear (Figure 5.2). The late hepatocyte marker is already expressed in undifferentiated RLEC cells. Its expression disappears on day 3 of the differentiation process and gradually re-appears from day 6 onwards (Figure 5.3). The cytoplasmic localisation of this factor indicates that it does not perform its function. However, from day 18 onwards, HNF-1alpha again translocates to the nucleus.

To conclude, the expression pattern of the above liver enriched transcription factors in RLEC undergoing differentiation indicates increasing maturation of the phenotype, since the expression of HNF-3beta and HNF-4, respectively, decreases and translocates to the cytoplasm (perinuclear), while the expression of the late HNF-1alpha increases in the nucleus.

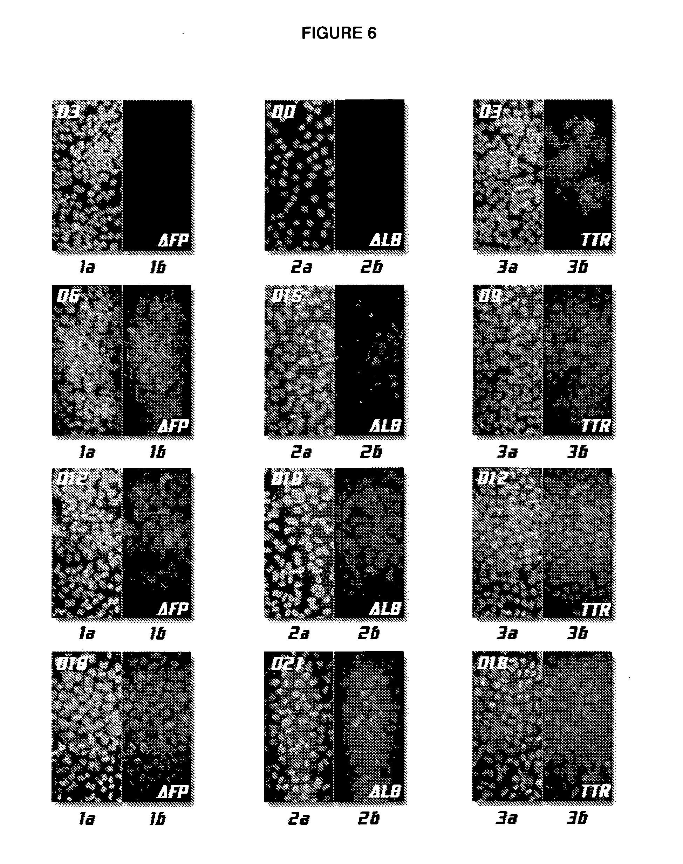

As known in the literature, HNF-3beta and HNF-1alpha regulate the expression of AFP and ALB, which are typical hepatic markers abundant in the foetal and adult liver, respectively. During differentiation of the RLEC cells, the expression of AFP increases from day 6 onwards, which is followed by downregulation on day 12 and again an upreguiation on day 18 of the procedure (Figure 6.1). In analogy with the *in vivo* situation, the expression of ALB takes place at a later stage, in particular from day 15 of the differentiation process onwards (Figure 6.2). Subsequently, a significant increase in ALB-expressing cells is observed (student t)test, p>0.001), with a maximum on day 21. TTR, also called prealbumin, is already observed in nuclei of undifferentiated RLEC and undergoes translocation to the cytoplasm from day 0 of the differentiation process onwards (Figure 6.3). Its expression remains substantially constant during the entire process (student t-test, p>0.05).

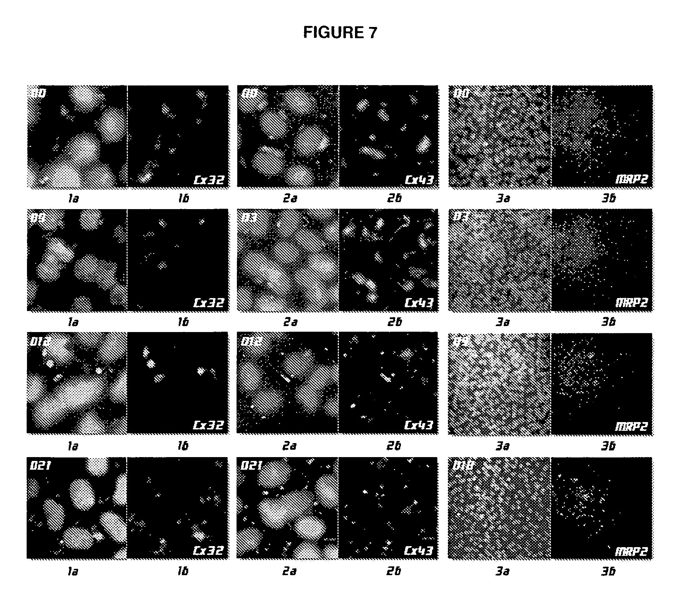

The connexins Cx32 and Cx43 are expressed, respectively, to a larger and a smaller degree during RLEC differentiation (Figure 7.1 and 7.2). Cx32 is not present in undifferentiated RLEC but shows gradual upregulation on the cell membrane during the differentiation (Figure 7.1). On the other hand, Cx43 is strongly expressed in undifferentiated cells. It expression increases during the first 3 days of the differentiation process and subsequently decreases, with partial migration of the cytoplasmic signal (perinuclear) to the cell membrane.

The expression of MRP2 is upregulated as of the onset of the differentiation process and remains substantially constant throughout (student t-test, p>0.05).

### Conclusions

The differentiation paradigm as detailed in example 5 stimulates differentiation of cells of an RLEC line towards hepatocyte-like cells, as evidence by several morphological and marker expression criteria.

In particular, during the progress of differentiation, the RLEC adopted polygonal to cubical morphology and starting from day 18, bi-nucleated cells could be observed.

Regarding the expression of marker proteins, the inventors speculate that the cells may be undergoing presumed 'dedifferentiation' early in the process (between days 0 and 3), as evidenced by decreasing expression of late liver enriched transcription factors and increase in Cx43. Without being limited to any hypothesis, the inventors presume that the RLEC may be further down a given liver developmental pathway and that the addition of FGF-4 may stimulate the cells to loose some of their partially differentiated properties. In view hereof, the inventors envisage differentiation protocols in which the initial step involving FGF-4 addition (as in example 5) is omitted (e.g., see protocols in example 6).

In this study, the actual differentiation towards the hepatic phenotype thus begins from day 3 onwards, with gradual decrease of expression of the biliary markers CK19 and Cx43, stable expression of TTR, CK18 and MRP2, and gradual increase in the expression of AFP, ALB and Cx32. Please also note that AFP is expressed early during the differentiation process, while ALB appears at a later stage, thus paralleling the *in vivo* situation.

### Example 8: Immunofluorescence protocol-staining on cultured cells

### Material

20% paraformaldehyde (PFA): 100g PFA EM-grade; PBS until 500ml; + 1.25ml 10N NaOH (= 40g NaOH in 100ml distilled water); heat at 60°C with magnetic stir barr until just dissolved; filter 0.22µm; cool on ice; adjust pH to 7.2 with HCl; aliquot per 2ml in 15ml tubes; store at - 20°C = 20% stock solution
4% PFÁ in PBS: add 8ml PBS to each 2ml 20% PFA; heat at 37°C with regular mixing until just dissolved (about 15 min); once dissolved, use immediately
50% Ethanol/50%Aceton: add 50ml absolute Ethanol to 50ml Aceton 100%
100mM glycine: 750mg glycin in 100ml PBS; filter 0.22µm; add 0.01 % sodium azide for storage; store at 4°C
10% sodium azide: 10g sodium azide in 100ml distilled water
PBST: PBS + 0.1% Triton X-100 = 0.5ml Triton X-100 in 500ml PBS
block buffer (1% BSA/5% donkey serum): 1g BSA (exclude when using goat primary Abs); 5ml normal donkey serum (block with normal serum from the same host species as the secondary, labeled antibody); 100ml PBS; filter 0.22µm; add 0.01% sodium azide for storage; aliquot per 2ml and freeze at -20°C; store working solution at 4°C

### Overview of antibodies:

| **Primary antibodies** | ***Type*** | ***Species*** | ***Dilution*** | ***Fixation*** |
|---|---|---|---|---|
| **AFP** Santa Cruz sc-8108 | Polyclonal | goat | 1/25-1/50 | PFA |
| **HNF-1** Santa Cruz sc-10791 | Polyclonal | rabbit | 1/25-1/50 | PFA |
| **CK18-FITC** Sigma F4772 | Monoclonal | mouse | 1/25 | EtOH/Aceton |
| **CK19** Sigma C7159 | Monoclonal | mouse | 1/20 | EtOH/Aceton |
| **ALB-FITC** Bethyl laboratories A90-234F | Polyclonal | goat | 1/100-1/200 | PFA |
| **MRP2** Santa Cruz sc-5770 | Polyclonal | goat | 1/50 | EtOH/Aceton |
| **Cx32** Sigma C3470 | Polyclonal | rabbit | 1/350-1/450 | EtOH |
| **Cx43** Sigma C6219 | Polyclonal | rabbit | 1/100 | EtOH |
| **TTR** Santa Cruz sc-13098 | Polyclonal | rabbit | 1/25-1/50 | PFA |
| **c-Kit** Santa Cruz; sc-1494 | Polyclonal | goat | 1/50 | PFA |
| **HNF-4α** Santa Cruz sc-8987 | Polyclonal | rabbit | 1/50 | PFA |
| **HNF-3β** Santa Cruz sc-6554 | Polyclonal | goat | 1/75 | PFA |

| **Secondary antibodies** | ***Specification*** | ***Species*** | ***Dilution*** |
|---|---|---|---|
| **anti-mouse IgG (H+L)-FITC** | Jackson Immunoresearch 715-095-150 | Donkey | 1/500 |
| **anti-goat IgG (H+L)-FITC** | Jackson Immunoresearch 705-096-147 | Donkey | 1/500 |
| **anti-goat IgG (H+L)-Cy-3** | Jackson Immunoresearch 705-166-147 | Donkey | 1/1500 |
| **anti-rabbit IgG (H+L)-FITC** | Jackson Immunoresearch 711-095-152 | Donkey | 1/500 |
| **anti-rabbit IgG (H+L)-Cy-3** | Jackson Immunoresearch 711-165-152 | Donkey | 1/1500 |

### Immunofluorescence

- rinse cells 3 x with PBS
- fix nuclear and cytoplasmic proteins at room temperature for 10min with 4%PFA (cold), connexins at -20°C for 10min with Ethanol/Aceton (50/50) and cytoskeletal proteins at -20°C for 10 min with Ethanol.
- wash 3 x 5min with PBS
- proceed with staining or store wells in PBS at 4°C
- check cells under microscope for autofluorescence and attachment
- incubate 15min with 100mM glycin in PBS (used to saturate reactive groups generated after PFA fixation)
- wash 3 x 5min with PBS
- incubate 15min with PBST (permeabilization)
- rinse 3 x with PBS
- block 45min with block buffer at room temperature
- incubate overnight at 4°C with primary Ab diluted in PBS
- rinse with PBS
- wash 4 x 15min with PBS
- incubate with fluorescence-labeled secondary Ab 1h30 at room temperature (dilute Ab in PBS)
- keep dark
- wash 4x15min with PBST
- rinse with distilled water
- mount with Vectashield with DAPI (use 3 drops per well)

### Quantity/well

- 200µl Abs / 2cm²-well
- 250µl block buffer / 2cm²-well
- 500µl PBS-PBST-glycin / 2cm²-well

### Example 9: Testing in vitro HDAC inhibition potencies of AN-7, 8, 9, 10, 13, 14, 15, and 16

A two-step collagenase perfusion technique was used to isolate hepatocytes from male outbred Sprague-Dawley rats (200-300g; Charles River Laboratories, Brussels, Belgium), which were kept under controlled environmental conditions (12h light-dark cycle) and fed a standard diet (Animalabo A 04) with water ad libitum. Procedures for housing of the animals, and for isolation and cultivation of the rat hepatocytes were approved by the local ethical committee of the Vrije Universiteit Brussel (VUB, Brussels, Belgium).

Inhibition of HDAC activity was assessed in crude rat hepatocyte lysates by the Fluor de lys HDAC inhibition protocol as outlined herein:
HDAC Fluorescent Activity Assay/Drug Discovery Kit, Tebu-bio, AK-500.

The developer and the substrate can be purchased separately:
- Fluor de Lys Developer concentrate 034KI-105-0300, 300µl
- Fluor de Lys substrate 034KI-104-0050, 50µl

Everything is stored in a box at -80°C.

Microtiter plates: 96-well, optical bottom, black, without lid, sterile, 10/pack, 0.4 ml (Nunc 265301), obtained from VWR international.

### Preparation of solutions:

- HDAC: thaw a rat hepatocyte cell extract (containing 6mg/ml proteins) in tap water at room temperature, store on ice.
- HDAC inhibitor:

### Prepare a dilution series in assay buffer.

The solvent control contains assay buffer + organic solvent concentration.
- Fluor de Lys substrate:
   Dilute the 50mM stock 100x (500µM) in assay buffer (for ex.: 2µl+198µl buffer)
   Dilute the 500µM stock 12.5x (40µM) in assay buffer (for ex: 100µl+1150µl buffer)

The total volume required = number of wells x 25µl (or 300µl/row)

Note: Km of rat hepatocyte HDAC towards the Fluor de Lys substrate was determined to be 10µM.
- Fluor de Lys developer:

### Prepare max. 30 min in advance!!

### Dilute the concentrate developer 20x in ice-cold assay buffer.

For max. 30 wells (2.5 rows): 75µl concentrated developer + 15µl TSA 0.2mM + 1410µl assay buffer.

### Store on ice until use.

Best is to aliquot developer upon receipt from Tebu-bio (4x75µl).
- Control of a potential effect of the inhibitor on the development and intensity of the fluorescence signal: spike with 5µM deacetylated standard (in the kit) after the 15 min incubation period. Wait another 15 min, add developer and measure fluorescence as usual. Compare the signal with the fluorescence of an equimolar concentration of deacetylated standard without inhibitor.

### Preparation of the assay buffer pH 8.0:

| | |
|---|---|
| 25mM Tris.HCl (Calbiochem 648317; MW 157.6) | 3.94 g/l |
| 137mM NaCl (Merck; MW 58.44) | 8.0063 g/l |
| 2.7mM KCI (Merck 0064859; MW 74.56) | 0.2013 g/l |
| 1mM MgCl₂ (.6H2O; Sigma M-2670; MW 203.3) | 0.2033 g/l |
| Bidest water | ad 1l |

Adjust the pH to 8.0 with HCl/NaOH 1-5M.

Store at 4°C during 6 months.

### Preparation of the rat hepatocyte cell extracts:

### Homogenisation buffer, pH 7.5 (adjust with 1 M HCl/NaOH):

Tris.HCl (MW 157.6) 50mM or 7.880 g/I
Sucrose (MW 342.3) 0.25mM or 0.0856g/l
KCI (MW 74.56) 25mM or 1.864 g/l
MgCl₂ (waterfree, MW 95.21) 5mM or 0.4760 g/I
Bidest water ad 1 I

### Work always on ice

- Add 500µl homogenisation buffer to 30x106 hepatocytes (pellets stored at -80°C).
- Ultrasonicate on ice 2 x 30 pulses
- Let the homogenate(s) rest on ice during 5 min.
- Transfer the homogenate(s) to 1.5ml eppendorf tube(s) on ice, vortex vigorously.
- Centrifugate at 14000rpm in the microcentrifuge during 5 min at 4°C.
- Determine the protein concentration (Bradford protein assay) of the supernatant, discard the pellets.
- Dilute the extract(s) to a protein concentration of 6mg/ml. Divide in aliquots of 500 µl (eppendorf tubes) and store for later use at -80°C.

### Procedure:

1. bring 10 µl inhibitor/control solvens/assay buffer per well
2. add 15 µl cell extract to each well (for the blank: 15 µl boiled cell extract)
3. incubate 5 min at room temperature (cover plate with aluminium foil)
4. add 25 µl Fluor de Lys substrate to each well (40µM solution, 10 µM end concentration)
5. cover the plate with aluminium foil and incubate 15 min on orbital shaker
6. after 15 min, add 50 µl freshly prepared, ice-cold developer solution to each well
7. cover the plate with aluminium foil and incubate 15 min on orbital shaker
8. Measure the fluorescence in the plate reader

### Evaluation of the results:

- Subtract the blank value (boiled extract) (background signal)
- Calculate as % of ethanol control, that is set 100%
- If the concentrations are spread around the 50% inhibition value, than the IC50 value can be calculated. If not, new dilutions of the compound have to be prepared and tested.
- IC50 values are determined using GraphPad Prism software, non-linear regression, ones-site competition. (Note: the concentration should be set out as their logarithmic values)

### Example 10:

### a) Inhibition of cell proliferation by HDAC inhibitors

Dose-dependent inhibition of cell proliferation by Jung-type HDACi after 3 days of incubation. Hepatocytes (0.4 x 10⁵ cells/cm²) were cultured in duplicate in a mixture of MEM/M199 (3:1, v/v) and stimulated to proliferate with EGF (50ng/ml) 4h after plating. Exposure to the indicated concentrations of HDACi started at time of plating and was maintained for 3 consecutive days. ³H-methylthymidine incorporation was measured at day 3 (72h) of culture. Cells cultured in the absence of EGF serve as a negative control for hepatocyte proliferation (neg. contr.) whereas cells stimulated with EGF alone serve as a positive control for hepatocyte proliferation (pos. contr.). Results are expressed as the percentage of maximal incorporation observed in hepatocytes cultured in the presence of EGF alone. Values shown are the means±SD of duplicate cultures of 3 experiments. The results are shown in Figure 12.

### b) Measurement of lactate dehydrogenase leakage into culture media

Hepatocytes (0.4 x 10⁵ cells/cm²) were cultured in duplicate in a mixture of MEM/M199 (3:1; v/v) and stimulated to proliferate with EGF (50ng/ml) 4h after plating. Exposure to the indicated concentrations of HDACi started at time of plating and was maintained for 3 consecutive days. At the time points indicated in the graphs, medium samples (500µl) were taken and immediately frozen in liquid nitrogen and stored at -80°C. The cells were scraped off in culture medium and immediately frozen in liquid nitrogen and stored at -80°C. Samples were thawed on ice and ultrasonicaled. LDH reagent was prepared as advised by manufacturer and placed in thermostated water bath at 25°C. 20µl of sample and 1ml of reagent were brought into microcuvette and mixed. The decrease in absorption was measured after 1 min during 2min. LDH activity was expressed in U/I and was calculated using the equation (A x F), A being the decrease in absorption/min and F correction factor (8095). The % LDH leakage was calculated as the result of the ratio: (100 x LDH activity in supernatant) / (LDH activity in supernatant+cells). The results are shown in Figure 13 (n=3).

### Example 11:

a) Effect of HDAC inhibitors on expression of cell cycle mediators in rat primary hepatocytes Hepatocytes (0.4 x 10⁵ cells/cm²) were cultured in duplicate in a mixture of MEM/M199 (3:1; v/v) and stimulated to proliferate with EGF (50ng/ml) 4h after plating. Exposure to the indicated concentrations of HDACi started at time of plating and was maintained for 3 consecutive days. Cells were then harvested and analyzed for cyclin D1 and cdk1 expression levels (20µg proteins/lane). Total ERK staining serves as a loading control. Results are shown in Figure 8.
b) Effect of HDAC inhibitors on expression of CYP450 isoforms in rat primary hepatocytes. Hepatocytes (0.57 x 10⁵ cells/cm²) were cultured in Williams' E medium supplemented with insulin (0.5µg/ml), glucagon (7ng/ml) and hydrocortisone (25µg/ml). Exposure to the indicated concentrations of HDACi started at time of plating and was maintained for 4 consecutive days. Cells were harvested at indicated time points and analyzed for CYP2 B1 and CYP1 A1 expression levels (40µg proteins/lane). Results are as presented in Figure 9.

### Example 12: Effect of TSA on the expression and activity of liver enriched transcription factors (LETFs) during hepatic differentiation of RLECs

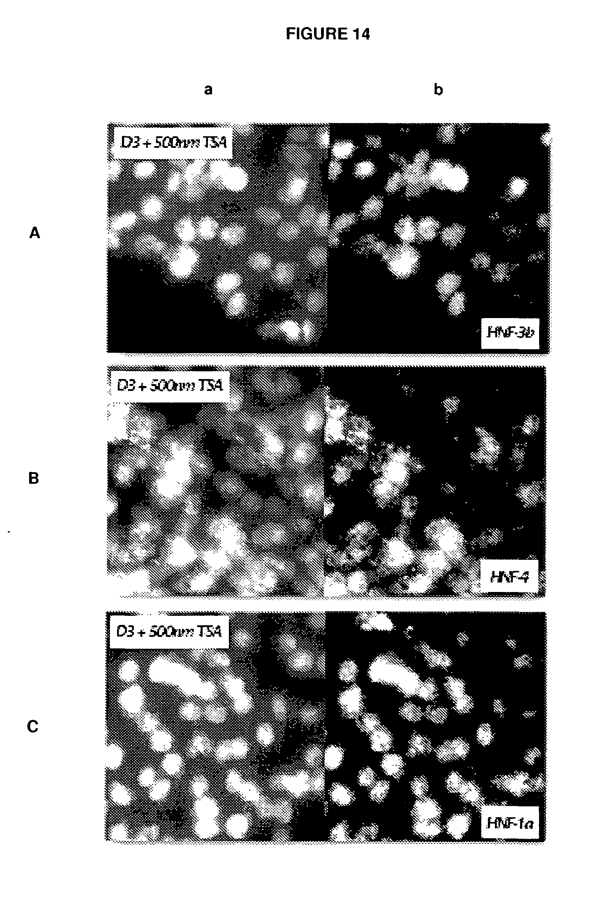

The inclusion of HDAC inhibitors, particularly HDAC-I inhibitors (in the present example TSA), within the current differentiation technique leads to an earlier expression and activity of hepatocyte nuclear factors (HNFs). Without the addition of TSA, the expression of the early LETF HNF-3β is stable during the first 12 days with a gradual increase in activity linked to the translocation from cytosol to the nucleus. With addition of TSA, HNF-3β is already expressed at D3 of the differentiation process and is located inside the nucleus. This indicates that HNF-3β is already active at this stage of hepatic differentiation (Figure 14A). The mid-late LETF HNF-4, which is not expressed by undifferentiated RLECs, is expressed and active from D3 onwards, either with or without addition of TSA, indicated by a nuclear localization (Figure 14B). When TSA is not present, the late LETF HNF-1α is not expressed at D3, but gets a gradual upregulation and translocation to the nucleus from D6 onwards. In comparison, addition of TSA to the culture medium induces expression of HNF-1α in nucleus already on D3 (Figure 14C).

It can be concluded that the addition of an HDAC inhibitor, herein TSA, has a clear influence on the expression and activity of hepatocyte nuclear factors during hepatic differentiation and maturation.

For this experiment, the protocols of example 5 were used to differentiate RLECs towards the hepatic lineage with the exception of the sequential procedure. Sequential 3 from example 6 was used to differentiate the cells instead of the one stated in example 5. The immunofluorescence protocol-staining on cultured cells was used as stated in example 8.

### Example 13: Morphology of RLEC differentiated in the presence of an HDAC inhibitor

RLEC are differentiated without initial FGF-4 exposure, according to the following sequence:
1. days 0-3: 20ng/ml HGF
2. days 3-6: 5ng/ml FGF-4, 30ng/ml HGF, 0.5 x ITS
3. days 6-9: 30ng/ml HGF, 0.25 x ITS; 0.05µM dexamethasone
4. days 9-12: 20ng/ml HGF, 0.05µM dexamethasone
5. days 12-15: 10ng/ml HGF; 10ng/ml OSM, 0.05µM dexamethasone
6. From day 15 on: 10ng/ml OSM, 0.05µM dexamethasone

TSA was added from D0 on every 3 days (when medium is changed).

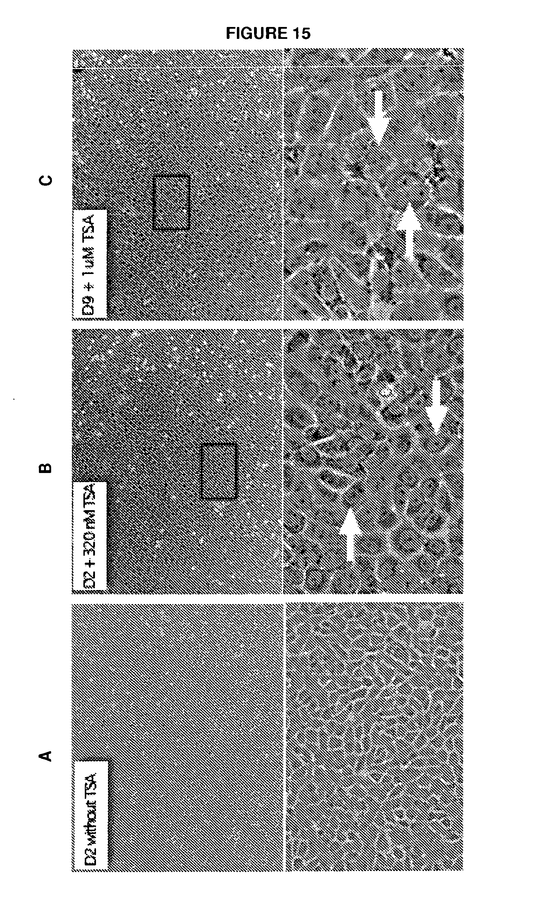

The results are illustrated in Figure 15. Figure 15A shows differentiating RLEC without addition of TSA, cultivated as above. The cells still have the characteristic RLEC shape. In contrast, Figure 15B shows differentiating RLEC on D2 after addition of 320nM TSA. The cells already display more polygonal shape (arrows), although binuclear cells seem not yet present. Figure 15C shows differentiating RLEC on D9 with the addition of 1µM TSA. Although this concentration of TSA causes some cell death, the cells differentiate into binuclear, polygonal to cuboidal shaped cells (arrows) already indicating hepatic maturation on D9. Optimisation of HDAC inhibitor concentration to reduce cell death is within the reach of a skilled person.

## Claims

1. A method for differentiating cells of a rat liver epithelial cell (RLEC) line to hepatocyte-like cells *in vitro,* comprising exposure of the cells of the RLEC line to two or more differentiation agents, wherein the cells are exposed sequentially to at least two of the said differentiation agents, and wherein two or more or all of the differentiation agents to which the cells of the RLEC line are exposed are chosen from fibroblast growth factor 4 (FGF-4), hepatocyte growth factor (HGF), insulin, transferrin, oncostatin M (OSM), or a biologically active variant or derivative of any of the above, selenium or a biologically acceptable acid or salt thereof, and a glucocorticoid.

2. The method according to claim 1, comprising:
(a) exposing the cells of the RLEC line to FGF-4 or a biologically active variant or derivative thereof during a time interval, the onset of which is between *t*=0 hours and *t*=72 hours, more preferably between *t*=0 hours and *t*=48 hours, even more preferably between *t*=0 hours and *t*=24 hours; and/or
(b) exposing the cells of the RLEC line to HGF or a biologically active variant or derivative thereof during a time interval, the onset of which is between *t*=0 hours and *t*=96, preferably between *t*=0 hours and *t*=48 hours, and more preferably between *t*=0 hours and *t*=24 hours; and/or
(c) exposing the cells of the RLEC line to insulin or a biologically active variant or derivative thereof during a time interval, the onset of which is between *t*=24 hours and *t*=240 hours, preferably between *t*=48 hours and *t*=192 hours, and more preferably between *t*=72 hours and *t*=144 hours; and/or
(d) exposing the cells of the RLEC line to transferrin or a biologically active variant or derivative thereof during a time interval, the onset of which is between *t*=24 hours and *t*=240 hours, preferably between *t*=48 hours and *t*=192 hours, and more preferably between *t*=72 hours and *t*=144 hours; and/or
(e) exposing the cells of the RLEC line to selenium or a biologically acceptable acid or salt thereof during a time interval, the onset of which is between *t*=24 hours and *t*=240 hours, preferably between *t*=48 hours and *t*=192 hours, and more preferably between *t*=72 hours and *t*=144 hours; and/or
(f) exposing the cells of the RLEC line to a glucocorticoid during a time interval, the onset of which is between *t*=72 hours and *t*=240 hours, preferably between *t*=96 hours and *t*=192 hours; and/or
(g) exposing the cells of the RLEC line to OSM or a biologically active variant or derivative thereof during a time interval the onset of which is between *t*=140 hours and *t*=480 hours, more preferably between *t*=264 hours and *t*=312 hours, even more preferably between *t*=276 hours and *t*=300 hours;
wherein *t*=0 hours is the starting time point of the said method.

3. The method according to claim 2, comprising at least the step (b) and any one, any two or all three of steps (c), (d) and (e).

4. The method according to claim 3, additionally comprising one or both of the steps (f) and (g).

5. The method according to any of claims 1 to 4, further comprising exposing the cells of the RLEC line to a histone deacetylase (HDAC) inhibitor.

6. The method according to claim 5, wherein the cells are exposed to the HDAC inhibitor during a time interval, the onset of which is between *t*=0 hours and *t*=96 hours.

7. The method according to claim 5, wherein the cells are exposed to the HDAC inhibitor during a time interval, the onset of which is between *t*=96 hours and *t*=480 hours, preferably at about *t*=144 hours or later.

8. The method according to any of claims 5 to 7, wherein said HDAC inhibitor is trichostatin A (TSA) or a salt thereof.

9. The method according to any of claims 5 to 7, wherein said HDAC inhibitor is chosen from compounds of formulas (I) to (XI) and salts thereof:

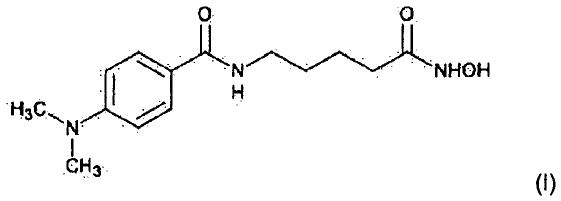

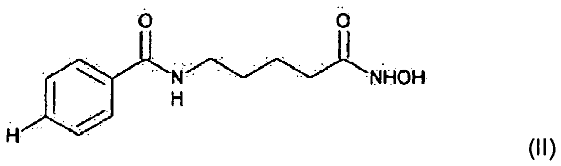

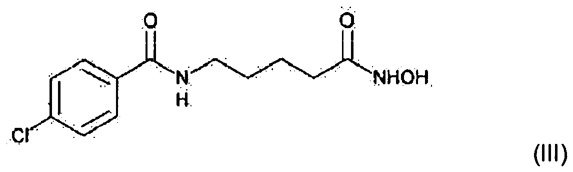

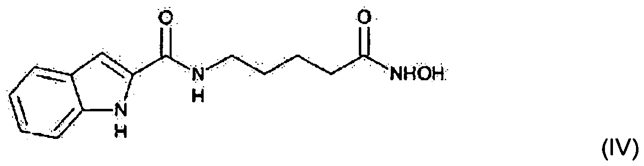

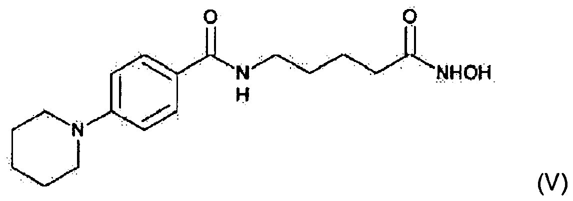

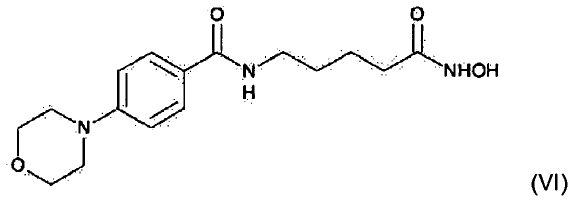

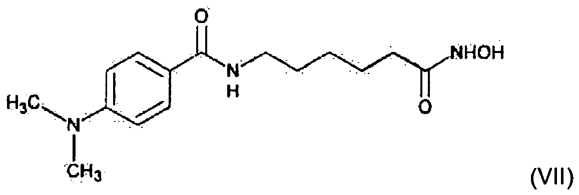

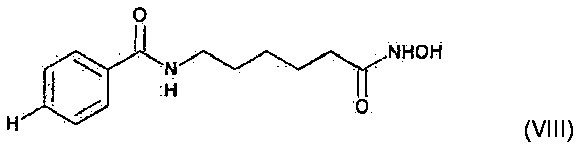

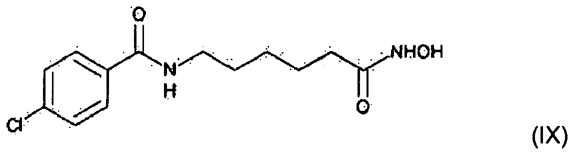

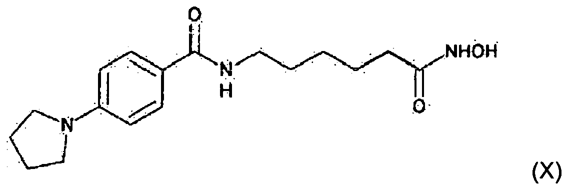

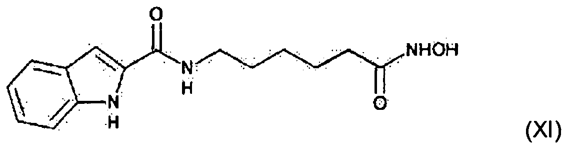

10. The method according to any one of claims 1 to 9, comprising exposure of the cells of the RLEC line to the two or more differentiation agents, as follows:
- days 0-1: FGF-4 or a biologically active variant or derivative thereof;
- days 1-3: FGF-4 and HGF, or a biologically active variant or derivative thereof;
- days 3-6: FGF-4, HGF, insulin and transferrin, or a biologically active variant or derivative thereof, and selenium or a biologically acceptable acid or salt thereof;
- days 6-9: HGF, insulin and transferrin, or a biologically active variant or derivative thereof, selenium or a biologically acceptable acid or salt thereof, and a glucocorticoid;
- days 9-12: HGF or a biologically active variant or derivative thereof and a glucocorticoid;
- days 12-15: HGF and OSM, or a biologically active variant or derivative thereof, and a glucocorticoid;
- from day 15 on: OSM or a biologically active variant or derivative thereof, and a glucocorticoid.

11. The method according to any one of claims 1 to 9, comprising exposure of the cells of the RLEC line to the two or more differentiation agents, as follows:
- days 0-3: HGF or a biologically active variant or derivative thereof;
- days 3-6: HGF, insulin and transferrin, or a biologically active variant or derivative thereof, and selenium or a biologically acceptable acid or salt thereof;
- days 6-9: HGF, insulin and transferrin, or a biologically active variant or derivative thereof, selenium or a biologically acceptable acid or salt thereof, and a glucocorticoid;
- days 9-12 HGF or a biologically active variant or derivative thereof and a glucocorticoid;
- days 12-15: HGF and OSM, or a biologically active variant or derivative thereof, and a glucocorticoid;
- from day 15 on: OSM or a biologically active variant or derivative thereof, and a glucocorticoid.

12. Use of rat hepatocyte-like cells or a cell population comprising such for the manufacture of a medicament for the treatment of liver diseases, wherein cells of RLEC line are exposed to two or more differentiation agents, wherein the cells of the RLEC line are exposed sequentially to at least two of the said differentiation agents, and wherein two or more or all of the differentiation agents to which the cells of the RLEC line are exposed are chosen from FGF-4, HGF, insulin, transferrin, OSM, or a biologically active variant or derivative of any of the above, selenium or a biologically acceptable acid or salt thereof, and a glucocorticoid, thereby differentiating the cells of the RLEC line to the hepatocyte-like cells *in vitro.*

13. Method for assaying toxicity, carcinogenicity or biotransformation in hepatocyte-like cells or a cell population comprising such, or method for preparation of bio-artificial liver devices or liver assist devices comprising hepatocyte-like cells or a cell population comprising such, comprising the method for differentiating cells of RLEC line to the hepatocyte-like cells *in vitro* as defined in any one of claims 1 to 9.

14. Method for providing a pharmaceutical composition comprising rat hepatocyte-like cells or a cell population comprising such, comprising the method for differentiating cells of RLEC line to the hepatocyte-like cells *in vitro* as defined is any one of claims 1 to 9.

15. The method according to any one of claims 1, 2 10 or 11, wherein the glucocorticoid is dexamethasone.

## Patentansprüche

1. Verfahren zum Differenzieren von Zellen einer Rattenleber-Epithelzellen(RLEC)-Linie zu Hepatozyten-artigen Zellen *in vitro,* umfassend das Exponieren der Zellen der RLEC-Linie gegenüber zwei oder mehr Differenzierungsmitteln, wobei die Zellen aufeinanderfolgend gegenüber wenigstens zwei der Differenzierungsmittel exponiert werden und wobei zwei oder mehr oder alle der Differenzierungsmittel, gegenüber denen die Zellen der RLEC-Linie exponiert werden, ausgewählt sind aus Fibroblasten-Wachstumsfaktor 4 (FGF-4), Hepatozyten-Wachstumsfaktor (HGF), Insulin, Transferrin, Oncostatin M (OSM) oder einer/einem biologisch aktiven Variante oder Derivat von einem der Vorstehenden, Selen oder einer/einem biologisch verträglichen Säure oder Salz davon und einem Glucocorticoid.

2. Verfahren gemäß Anspruch 1, umfassend:
(a) Exponieren der Zellen der RLEC-Linie gegenüber FGF-4 oder einer/einem biologisch aktiven Variante oder Derivat davon über ein Zeitintervall, dessen Beginn zwischen *t* = 0 Stunden und *t* = 72 Stunden, bevorzugter zwischen *t* = 0 Stunden und *t* = 48 Stunden, noch bevorzugter zwischen *t* = 0 Stunden und *t* = 24 Stunden liegt; und/oder
(b) Exponieren der Zellen der RLEC-Linie gegenüber HGF oder einer/einem biologisch aktiven Variante oder Derivat davon über ein Zeitintervall, dessen Beginn zwischen *t* = 0 Stunden und *t* = 96, vorzugsweise zwischen *t* = 0 Stunden und *t* = 48 Stunden, bevorzugter zwischen t = 0 Stunden und *t* = 24 Stunden liegt; und/oder
(c) Exponieren der Zellen der RLEC-Linie gegenüber Insulin oder einer/einem biologisch aktiven Variante oder Derivat davon über ein Zeitintervall, dessen Beginn zwischen *t* = 24 Stunden und *t* = 240 Stunden, vorzugsweise zwischen *t* = 48 Stunden und *t* = 192 Stunden, bevorzugter zwischen *t* = 72 Stunden und *t* = 144 Stunden liegt; und/oder
(d) Exponieren der Zellen der RLEC-Linie gegenüber Transferrin oder einer/einem biologisch aktiven Variante oder Derivat davon über ein Zeitintervall, dessen Beginn zwischen *t* = 24 Stunden und *t* = 240 Stunden, vorzugsweise zwischen *t* = 48 Stunden und *t* = 192 Stunden, bevorzugter zwischen *t* = 72 Stunden und *t* = 144 Stunden liegt; und/oder
(e) Exponieren der Zellen der RLEC-Linie gegenüber Selen oder einer/einem biologisch verträglichen Säure oder Salz davon über ein Zeitintervall, dessen Beginn zwischen *t* = 24 Stunden und *t* = 240 Stunden, vorzugsweise zwischen *t* = 48 Stunden und *t* = 192 Stunden, bevorzugter zwischen *t* = 72 Stunden und *t* = 144 Stunden liegt; und/oder
(f) Exponieren der Zellen der RLEC-Linie gegenüber einem Glucocorticoid über ein Zeitintervall, dessen Beginn zwischen *t* = 72 Stunden und *t* = 240 Stunden, vorzugsweise zwischen *t* = 96 Stunden und *t* = 192 Stunden liegt; und/oder
(g) Exponieren der Zellen der RLEC-Linie gegenüber OSM oder einer/einem biologisch aktiven Variante oder Derivat davon über ein Zeitintervall, dessen Beginn zwischen *t* = 140 Stunden und *t* = 480 Stunden, bevorzugter zwischen *t* = 264 Stunden und *t* = 312 Stunden, noch bevorzugter zwischen *t* = 276 Stunden und *t* = 300 Stunden liegt;
wobei *t* = 0 Stunden der Startzeitpunkt des Verfahrens ist.

3. Verfahren gemäß Anspruch 2, umfassend wenigstens Schritt (b) und einen beliebigen, beliebige zwei oder alle drei der Schritte (c), (d) und (e).

4. Verfahren gemäß Anspruch 3, zusätzlich umfassend einen oder beide der Schritte (f) und (g).

5. Verfahren gemäß einem der Ansprüche 1 bis 4, ferner umfassend das Exponieren der Zellen der RLEC-Linie gegenüber einem Histondeacetylase(HDAC)-Hemmer.

6. Verfahren gemäß Anspruch 5, wobei die Zellen während eines Zeitintervalls, dessen Beginn zwischen *t* = 0 Stunden und *t* = 96 Stunden liegt, gegenüber dem HDAC-Hemmer exponiert werden.

7. Verfahren gemäß Anspruch 5, wobei die Zellen während eines Zeitintervalls, dessen Beginn zwischen *t* = 96 Stunden und *t* = 480 Stunden liegt, vorzugsweise bei etwa *t* = 144 Stunden oder später, gegenüber dem HDAC-Hemmer exponiert werden.

8. Verfahren gemäß einem der Ansprüche 5 bis 7, wobei der HDAC-Hemmer Trichostatin A (TSA) oder ein Salz davon ist.

9. Verfahren gemäß einem der Ansprüche 5 bis 7, wobei der HDAC-Hemmer ausgewählt ist aus Verbindungen der Formeln (I) bis (XI) und Salzen davon:

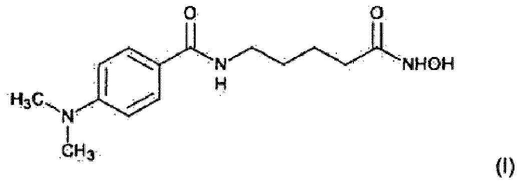

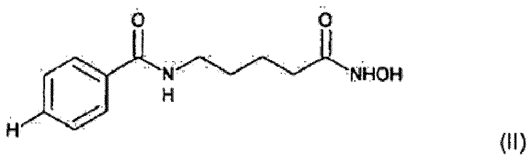

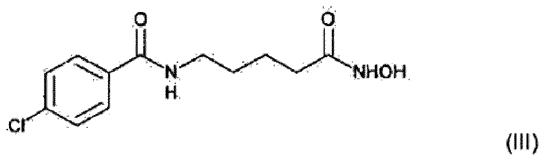

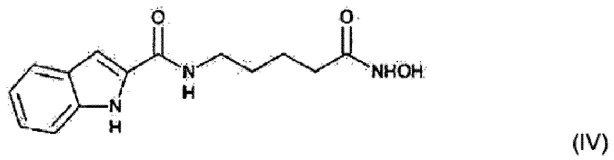

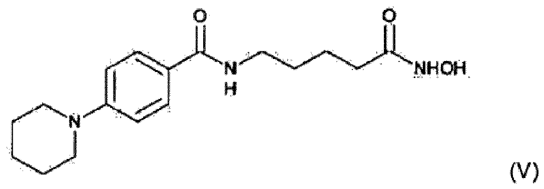

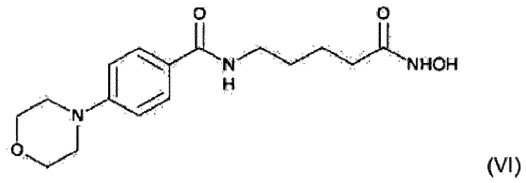

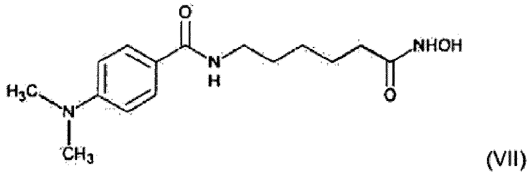

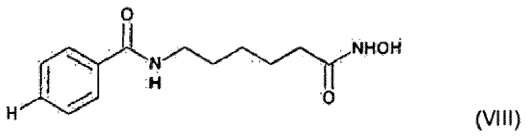

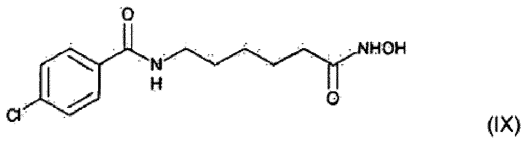

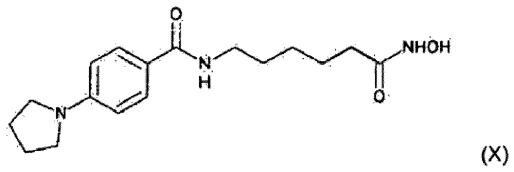

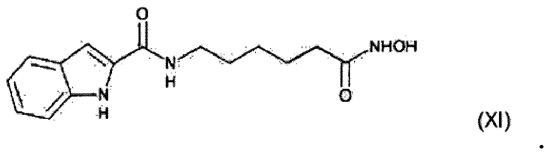

10. Verfahren gemäß einem der Ansprüche 1 bis 9, umfassend das Exponieren der Zellen der RLEC-Linie gegenüber den zwei oder mehr Differenzierungsmitteln wie folgt:
- Tage 0-1: FGF-4 oder ein(e) biologisch aktive(s) Variante oder Derivat davon;
- Tage 1-3: FGF-4 und HGF oder ein(e) biologisch aktive(s) Variante oder Derivat davon;
- Tage 3-6: FGF-4, HGF, Insulin und Transferrin oder ein(e) biologisch aktive(s) Variante oder Derivat davon und Selen oder ein(e) biologisch verträgliche(s) Säure oder Salz davon;
- Tage 6-9: HGF, Insulin und Transferrin oder ein(e) biologisch aktive(s) Variante oder Derivat davon, Selen oder ein(e) biologisch verträgliche(s) Säure oder Salz davon und ein Glucocorticoid;
- Tage 9-12: HGF oder ein(e) biologisch aktive(s) Variante oder Derivat davon und ein Glucocorticoid;
- Tage 12-15: HGF und OSM oder ein(e) biologisch aktive(s) Variante oder Derivat davon und ein Glucocorticoid;
- ab Tag 15: OSM oder ein(e) biologisch aktive(s) Variante oder Derivat davon und ein Glucocorticoid.

11. Verfahren gemäß einem der Ansprüche 1 bis 9, umfassend das Exponieren der Zellen der RLEC-Linie gegenüber den zwei oder mehr Differenzierungsmitteln wie folgt:
- Tage 0-3: HGF oder ein(e) biologisch aktive(s) Variante oder Derivat davon;
- Tage 3-6: HGF, Insulin und Transferrin oder ein(e) biologisch aktive(s) Variante oder Derivat davon und Selen oder ein(e) biologisch verträgliche(s) Säure oder Salz davon;
- Tage 6-9: HGF, Insulin und Transferrin oder ein(e) biologisch aktive(s) Variante oder Derivat davon, Selen oder ein(e) biologisch verträgliche(s) Säure oder Salz davon und ein Glucocorticoid;
- Tage 9-12: HGF oder ein(e) biologisch aktive(s) Variante oder Derivat davon und ein Glucocorticoid;
- Tage 12-15: HGF und OSM oder ein(e) biologisch aktive(s) Variante oder Derivat davon und ein Glucocorticoid;
- ab Tag 15: OSM oder ein(e) biologisch aktive(s) Variante oder Derivat davon und ein Glucocorticoid.

12. Verwendung von Rattenhepatozyten-artigen Zellen oder einer Zellpopulation, die solche enthält, für die Herstellung eines Medikaments für die Behandlung von Lebererkrankungen, wobei Zellen der RLEC-Linie gegenüber zwei oder mehr Differenzierungsmitteln exponiert werden, wobei die Zellen der RLEC-Linie aufeinanderfolgend gegenüber wenigstens zwei der Differenzierungsmittel exponiert werden und wobei zwei oder mehr oder alle der Differenzierungsmittel, gegenüber denen die Zellen der RLEC-Linie exponiert werden, ausgewählt sind aus FGF-4, HGF, Insulin, Transferrin, OSM oder einer/einem biologisch aktiven Variante oder Derivat von einem der Vorstehenden, Selen oder einer/einem biologisch verträglichen Säure oder Salz davon und einem Glucocorticoid, um so die Zellen der RLEC-Linie zu den Hepatozyten-artigen Zellen *in vitro* zu differenzieren.

13. Verfahren zum Untersuchen von Toxizität, Karzinogenität oder Biotransformation in Hepatozyten-artigen Zellen oder einer Zellpopulation, die solche umfasst, oder Verfahren zum Herstellen von bio-künstlichen Lebereinheiten oder Leberunterstützungseinheiten, umfassend Hepatozyten-artige Zellen oder eine Zellpopulation, die solche umfasst, umfassend das Verfahren zum Differenzieren von Zellen einer RLEC-Linie zu den Hepatozyten-artigen Zellen *in vitro* gemäß einem der Ansprüche 1 bis 9.

14. Verfahren zum Bereitstellen einer pharmazeutischen Zusammensetzung, umfassend Rattenhepatozyten-artige Zellen oder eine Zellpopulation, die solche umfasst, umfassend das Verfahren zum Differenzieren von Zellen einer RLEC-Linie zu den Hepatozyten-artigen Zellen *in vitro* gemäß einem der Ansprüche 1 bis 9.

15. Verfahren gemäß einem der Ansprüche 1, 2, 10 oder 11, wobei das Glucocorticoid Dexamethason ist.

## Revendications

1. Procédé de différenciation de cellules d'une lignée de cellules épithéliales hépatiques de rat (RLEC) en cellules hépatocyte-like *in vitro,* comprenant l'exposition des cellules de la lignée RLEC à deux ou plus agents de différenciation, les cellules étant exposées successivement à au moins deux desdits agents de différenciation, et deux, ou plus, ou la totalité des agents de différenciation auxquels les cellules de la lignée RLEC sont exposées étant choisis parmi le facteur de croissance des fibroblastes 4 (FGF-4), le facteur de croissance des hépatocytes (HGF), l'insuline, la transferrine, l'oncostatine M (OSM), ou un variant ou un dérivé biologiquement actif de l'un quelconque de ceux-ci, le sélénium ou un acide ou un sel biologiquement acceptable de ce dernier, et un glucocorticoïde.

2. Procédé selon la revendication 1, comprenant :
(a) l'exposition des cellules de la lignée RLEC au FGF-4 ou à un variant ou à dérivé biologiquement actif de ce dernier, pendant un certain laps de temps, dont le début se situe entre *t*=0 heure et *t*=72 heures, plus particulièrement entre *t*=0 heure et *t*=48 heures, d'une manière encore plus préférée entre *t*=0 heure et *t*=24 heures ; et/ou
(b) l'exposition des cellules de la lignée RLEC au HGF ou à un variant ou à un dérivé biologiquement actif de ce dernier pendant un certain laps de temps, dont le début se situe entre *t*=0 heure et *t*=96 heures, de préférence entre *t*=0 heure et *t*=48 heures, et plus particulièrement entre *t*=0 heure et *t*=24 heures ; et/ou
(c) l'exposition des cellules de la lignée RLEC à l'insuline ou à un variant ou à un dérivé biologiquement actif de cette dernière pendant un certain laps de temps, dont le début se situe entre *t*=24 heures et *t*=240 heures, de préférence entre *t*=48 heures et *t*=192 heures, et plus particulièrement entre *t*=72 heures et *t*=144 heures ; et/ou
(d) l'exposition des cellules de la lignée RLEC à la transferrine ou à un variant ou un dérivé biologiquement actif de cette dernière pendant un certain laps de temps, dont le début se situe entre *t*=24 heures et *t*=240 heures, de préférence entre *t*=48 heures et *t*=192 heures, et plus particulièrement entre *t*=72 heures et *t*=144 heures ; et/ou
(e) l'exposition des cellules de la lignée RLEC au sélénium ou à un acide ou un sel biologiquement acceptable de ce dernier pendant un certain laps de temps, dont le début se situe entre *t*=24 heures et *t*=240 heures, de préférence entre *t*=48 heures et t=192 heures, et plus particulièrement entre *t*=72 heures et *t*=144 heures ; et/ou
(f) l'exposition des cellules de la lignée RLEC à un glucocorticoïde pendant un certain laps de temps, dont le début se situe entre *t*=72 heures et *t*=240 heures, de préférence entre *t*=96 heures et *t*=192 heures ; et/ou
(g) l'exposition des cellules de la lignée RLEC à l'OSM ou à un variant ou à un dérivé biologiquement actif de cette dernière pendant un certain laps de temps, dont le début se situe entre *t*=140 heures et *t*=480 heures, de préférence entre *t*=264 heures et *t*=312 heures, d'une manière encore plus préférée entre *t*=276 heures et *t*=300 heures ;
où *t*=0 heure est l'instant du début dudit procédé.

3. Procédé selon la revendication 2, comprenant au moins l'étape (b) et l'une quelconque, deux quelconques des étapes (c), (d) et (e), ou les trois.

4. Procédé selon la revendication 3, comprenant en outre l'une des étapes (f) et (g), ou les deux.

5. Procédé selon l'une quelconque des revendications 1 à 4, comprenant en outre l'exposition des cellules de la lignée RLEC à un inhibiteur de l'histone désacétylase (HDAC).

6. Procédé selon la revendication 5, dans lequel les cellules sont exposées à l'inhibiteur de la HDAC pendant un certain laps de temps, dont le début se situe entre *t*=0 heure et *t*=96 heures.

7. Procédé selon la revendication 5, dans lequel les cellules sont exposées à l'inhibiteur de la HDAC pendant un certain laps de temps, dont le début se situe entre *t*=96 heures et *t*=480 heures, de préférence à environ *t*=144 heures, ou plus tard.

8. Procédé selon l'une quelconque des revendications 5 à 7, dans lequel ledit inhibiteur de la HDAC est la trichostatine A (TSA) ou un sel de cette dernière.

9. Procédé selon l'une quelconque des revendications 5 à 7, dans lequel ledit inhibiteur de la HDAC est choisi parmi les composés de formule (I) à (XI) et les sels de ces derniers :

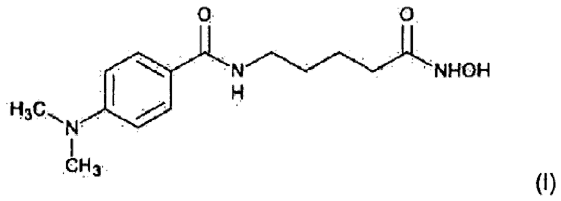

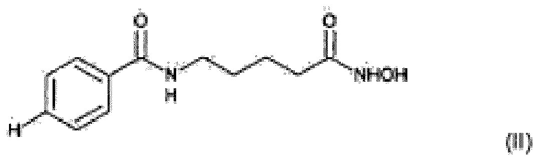

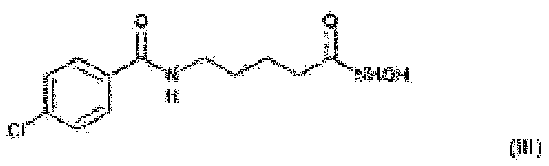

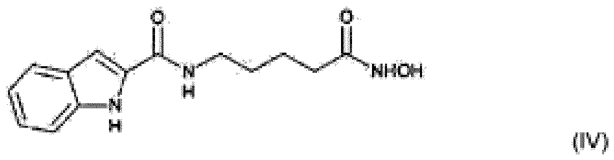

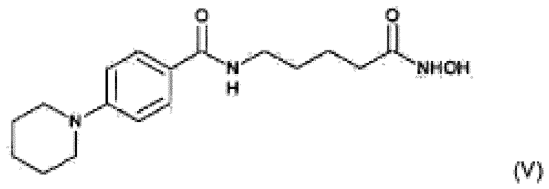

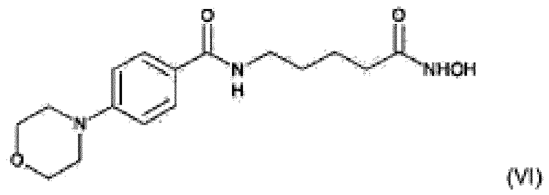

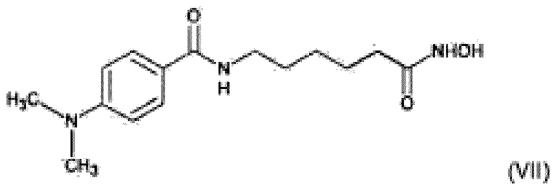

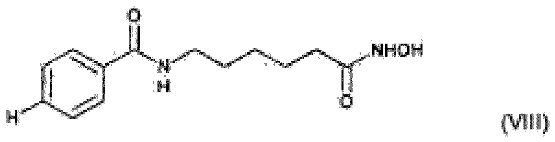

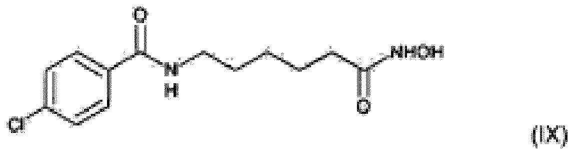

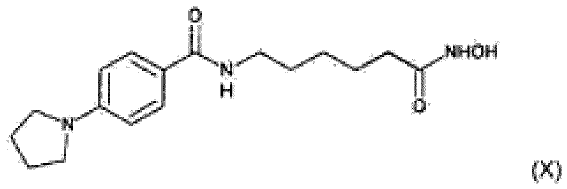

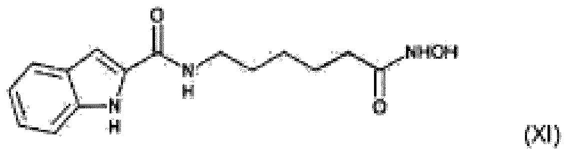

10. Procédé selon l'une quelconque des revendications 1 à 9, comprenant l'exposition des cellules de la lignée RLEC aux deux ou plus agents de différenciation, comme suit :
- jours 0-1 : FGF-4 ou un variant ou un dérivé biologiquement actif de ce dernier ;
- jours 1-3 : FGF-4 et HGF, ou un variant ou un dérivé biologiquement actif de ces derniers ;
- jours 3-6 : FGF-4, HGF, insuline et transferrine, ou un variant ou un dérivé biologiquement actif de ces derniers, et sélénium ou un acide ou un sel biologiquement acceptable de ce dernier ;
- jours 6-9 : HGF, insuline et transferrine, ou un variant ou un dérivé biologiquement actif de ces derniers, sélénium ou un acide ou un sel biologiquement acceptable de ce dernier, et un glucocorticoïde ;
- jours 9-12 : HGF ou un variant ou un dérivé biologiquement actif de ce dernier et un glucocorticoïde ;
- jours 12-15 : HGF et OSM, ou un variant ou un dérivé biologiquement actif de ces derniers, et un glucocorticoïde ;
- à partir du jour 15 : OSM ou un variant ou un dérivé biologiquement actif de cette dernière, et un glucocorticoïde.

11. Procédé selon l'une quelconque des revendications 1 à 9, comprenant l'exposition des cellules de la lignée RLEC auxdits deux ou plus agents de différenciation, comme suit :
- jours 0-3 : HGF ou un variant ou un dérivé biologiquement actif de ce dernier ;
- jours 3-6 : HGF, insuline et transferrine, ou un variant ou un dérivé biologiquement actif de ces derniers, et sélénium ou un acide ou un sel biologiquement acceptable de ce dernier ;
- jours 6-9 : HGF, insuline et transferrine, ou un variant ou un dérivé biologiquement actif de ces derniers, sélénium ou un acide ou un sel biologiquement acceptable de ce dernier, et un glucocorticoïde ;
- jours 9-12 : HGF ou un variant ou un dérivé biologiquement actif de ce dernier et un glucocorticoïde ;
- jours 12-15 : HGF et OSM, ou un variant ou un dérivé biologiquement actif de ces derniers, et un glucocorticoïde ;
- à partir du jour 15 : OSM ou un variant ou un dérivé biologiquement actif de cette dernière, et un glucocorticoïde.

12. Utilisation de cellules hépatocyte-like de rat ou d'une population cellulaire les comprenant, pour la fabrication d'un médicament pour le traitement des maladies hépatiques, pour laquelle des cellules de la lignée RLEC sont exposées à deux ou plus agents de différenciation, pour laquelle les cellules de la lignée RLEC sont exposées successivement à au moins deux desdits agents de différenciation, et pour laquelle deux ou plus ou la totalité des agents de différenciation auxquels les cellules de la lignée RLEC sont exposées sont choisis parmi le FGF-4, le HGF, l'insuline, la transferrine, l'OSM ou un variant ou un dérivé biologiquement actif de l'un quelconque d'entre eux, le sélénium ou un acide ou un sel biologiquement acceptable de ce dernier, et un glucocorticoïde, de façon à différencier les cellules de la lignée RLEC en les cellules hépatocyte-like *in vitro.*

13. Procédé d'évaluation de la toxicité, de la carcinogénicité ou de la biotransformation dans des cellules hépatocyte-like ou d'une population cellulaire les comprenant, ou procédé de préparation de dispositifs de foie bioartificiel ou de dispositifs d'assistance hépatique comprenant des cellules hépatocyte-like ou une population cellulaire les comprenant, comprenant le procédé de différenciation des cellules de la lignée RLEC en les cellules hépatocyte-like *in vitro,* tel que défini dans l'une quelconque des revendications 1 à 9.

14. Procédé pour mettre à disposition une composition pharmaceutique comprenant des cellules hépatocyte-like de rat ou une population cellulaire les comprenant, comprenant le procédé de différenciation de cellules de la lignée RLEC en les cellules hépatocyte-like *in vitro,* tel que défini dans l'une quelconque des revendications 1 à 9.

15. Procédé selon l'une quelconque des revendications 1, 2, 10 ou 11, dans lequel le glucocorticoïde est la dexaméthasone.
